# EUROPEAN PATENT SPECIFICATION

(11) **EP 2 256 188 B1**
(45) Date of publication and mention of the grant of the patent: **18.06.2014**
(21) Application number: 09714443.0
(22) Date of filing: 27.02.2009
(51) Int. Cl.: C12N 9/00, C12N 15/09, C12Q 1/68

(54) **METHOD FOR INCREASING ENZYMATIC REACTIVITY**
VERFAHREN ZUR ERHÖHUNG DER ENZYMATISCHEN REAKTIVITÄT
PROCÉDÉ D'AUGMENTATION DE LA RÉACTIVITÉ ENZYMATIQUE

(30) Priority: 29.02.2008 JP 2008050744
(43) Date of publication of application: 01.12.2010
(73) Proprietor: Riken, Wako-shi, Saitama 351-0198 (JP); Dnaform, Kanagawa 230-0046 (JP)
(72) Inventor: KOJIMA, Miki, Yokohama-shi Kanagawa 230-0045 (JP); CARNINCI, Piero, Wako-shi Saitama 351-0198 (JP); HAYASHIZAKI, Yoshihide, Yokohama-shi Kanagawa 230-0045 (JP); HARBERS, Matthias, Yokohama-shi Kanagawa 230-0046 (JP)
(74) Representative: Godemeyer Blum Lenze - werkpatent
(86) International application number: PCT/JP2009/053772
(87) International publication number: WO 2009/107816

(56) References cited:
- EP-A1- 1 072 678
- EP-A1- 1 712 618
- WO-A1-2005/030958
- WO-A1-2006/051990
- JP-A- 7 194 378
- JP-A- 10 084 954
- JP-A- 2005 118 036
- JP-T- 2000 514 298
- JP-T- 2005 535 311
- HALLING PETER J ET AL: "Understanding enzyme action on immobilised substrates.", CURRENT OPINION IN BIOTECHNOLOGY AUG 2005 LNKD- PUBMED:16005203, vol. 16, no. 4, August 2005 (2005-08), pages 385-392, XP002661028, ISSN: 0958-1669
- A SPIESS ET AL: "A Highly Efficient Method for Long-Chain cDNA Synthesis Using Trehalose and Betaine", ANALYTICAL BIOCHEMISTRY, vol. 301, no. 2, 15 February 2002 (2002-02-15), pages 168-174, XP55009246, ISSN: 0003-2697, DOI: 10.1006/abio.2001.5474
- CHEN L. ET AL.: 'Trehalose as a good candidate for enriching full-length cDNAs in cDNA library construction' J.BIOTECHNOL. vol. 127, no. 3, 2007, pages 402 - 407, XP005787032
- DIEHL F. ET AL.: 'BEAMing: single-molecule PCR on microparticles in water-in-oil emulsions' NAT. METHOD. vol. 3, no. 7, 2006, pages 551 - 559, XP002510523
- GEKKO K.: 'Why are proteins stable in the presence of sugars and polyols?' TANPAKUSHITSU KAKUSAN KOSO vol. 30, no. 10, 1985, pages 1115 - 1126, XP008135316

## Description

### [Technical Field]

The present invention relates to a method for reducing or suppressing the inhibitory effect of a support on the reactivity of an enzyme to a target substance using one or more substances selected from the group consisting of saccharides, amino acids, polyhydric alcohols, and derivatives thereof.

### Background Art

The method of synthesizing target DNA from a DNA or RNA template using biotin-labeled primer and purifying and recovering the target DNA obtained with paramagnetic streptavidin beads has been widely employed to purify and recover DNA. This method utilizes the stable, specific binding properties of streptavidin and biotin to efficiently recover highly pure cDNA by simply washing and magnetically aggregating the beads. Accordingly, one can expect to be able to efficiently prepare a highly pure cDNA library by this method. One can anticipate efficient sequencing when this method is applied to a sequencing reaction. It is also possible to apply this method to gene signature cloning (GSC), which yields considerable cDNA information in a single sequencing reaction that minimizes the 5' end and 3' end of cDNA. GSC was established by the present inventors (see Kojima et al., Collected Summaries of Presentations at the Jointly Convened 30th Annual Meeting of the Molecular Biology Society of Japan and 80th Meeting of the Japanese Biochemical Society, p. 495.

Various methods of increasing enzymatic activity in enzymatic reactions have been attempted to increase the target DNA recovery rate. In particular, the method of activating enzymes at high temperature in enzymatic reactions in uniform liquid phase systems is known. Based on this method, the presence of a substance functioning as a chaperone, such as sarcosine, betaine, or a sugar such as trehalose, activates enzymes at elevated temperatures (see Japanese Patent Nos. 3,206,894 and 3,536,052; Piero Caminci et al., (1998), Proc. Natl. Acad. Sci. USA, Vol. 95, Issue 2, 520-524; Y. Nagasawa et al., (2003), Cryobio, Cryotech. 49, 87-95; and Lei Chena, b, 1 et al., (2007), Journal of Biotechnology, 127, 402-407).

The document EP 1 712 618 A1 discloses a primer set comprising at least two primers that allows a target nucleic acid sequence to be amplified, wherein at least one primer included in the primer set has a solid-phase support or a site that can bind to a solid-phase support. Further, methods are disclosed wherein nucleic acids are amplified using a polymerase and at least one primer bound to a solid support, in the presence of an enzyme stabilizing agent (e.g. trehalose, sorbitol, mannitol) for improving the nucleic acid synthesis efficiency of and amplification efficiency.

### [Disclosure of the Invention]

The present inventors applied the purification and recovery of target DNA using paramagnetic streptavidin beads to the GSC method and successfully recovered target DNA in a simple and rapid manner. However, the quantity of target DNA recovered was much lower than what would have been expected in a uniform liquid phase system. The present inventors applied a target DNA purification and recovery method employing not beads but streptavidin plates to a DNA synthesis reaction. In the same manner as when employing a bead system, they were unable to recover target DNA in ample quantities relative to what one would expect from a uniform liquid phase system.

Based on these results, the present inventors considered that in an enzymatic reaction in which target DNA immobilized on a support such as beads or plates is subjected to the action of an enzyme, the enzymatic reaction may not fully take place, thereby lowering the recovery rate of target DNA. They also conceived that it might be possible to increase the recovery rate of target DNA under the above conditions by increasing the reactivity of the enzyme to the target DNA immobilized on a carrier such as beads or plates, or by reducing or suppressing the inhibitory effect of the support on the enzymatic reaction.

However, there was no known method of reducing or suppressing the inhibitory effect of a support on an enzymatic reaction.

Accordingly, the present invention has as its object to provide a method for reducing or suppressing the inhibitory effect of a support on an enzyme reaction.

### [Means of Solving the Problem]

The present inventors conducted extensive research into achieving the above-stated object. This resulted in the discovery that in a system where an enzyme was reacted with a target substance immobilized on a support, the addition of one or more substances selected from the group consisting of saccharides such as trehalose, amino acids, polyhydric alcohols, and derivatives thereof enhanced the reactivity of the enzyme. Based on this discovery, the present inventors prepared a cDNA library on a support, reacted it with a restriction enzyme, conducted a DNA synthesis reaction, and implemented the above GSC method in the presence of this substance, successfully improving the enzymatic reaction and purifying and recovering target DNA at a high recovery rate in all cases. Further, when the present inventors added this compound to the reaction solutions of Solexa sequencing systems implementing sequencing reactions and template amplification reactions on ultra-high density flow cells such as DNA microarrays, and to the reaction solutions of 454 sequencing systems employing emulsion PCR conducted by complementarily interacting DNA fragments with oligoprimer immobilized on beads in water-in-oil emulsions, they successfully increased the reaction efficiency. Accordingly, the present invention was devised on the basis of the above discovery.

That is, the present invention provides the following methods:
(1) A method for reducing or suppressing the inhibitory effect of a support, on which a target substance is immobilized, on the reactivity of an enzyme to the target substance by placing the enzyme in the presence of one or more substances selected from the group consisting of saccharides, amino acids, polyhydric alcohols.
(2) The method according to (1) wherein the sugar is selected from the group consisting of trehalose, maltose, glucose, sucrose, lactose, xylobiose, agarobiose, cellobiose, levanbiose, quitobiose, 2-β-glucuronosylglucuronic acid, allose, altrose, galactose, gulose, idose, mannose, talose, sorbitol, levulose, xylitol and arabitol.
(3) The method according to (1) or (2) wherein the amino acid is selected from the group consisting of N^{e}-acetyl-β-lysine, alanine, *gamma-*aminobutyric acid, betaine, glycine betaine, N^{a}-carbamoyl-L-glutamine-1-amide, choline, dimethylthetine, ecotine, glutamate, β-glutamine, glycine, octopine, proline, sarcosine, taurine, and trimethylamine N-oxide.
(4) The method according to any one of (1) to (3) wherein the target substance is a nucleic acid.
(5) The method according to (4) wherein the nucleic acid is a single-stranded or double-stranded DNA or RNA.
(6) The method according to (4) wherein the nucleic acid is comprised of hybridized DNA and RNA.
(7) The method of any one of (1) to (6) wherein the enzyme is one or more enzymes selected from the group consisting of transferase, hydrolase, and synthase.
(8) The method according to any one of (1) to (7) wherein the enzyme is DNA polymerase, RNase, DNA ligase and reverse transcriptase.
(9) The method according to any one of (1) to (6) wherein the enzyme is a restriction enzyme.
(10) The method according to any one of (1) to (9) wherein the support is a bead-like support or a plate-like support.
(11) The method according to (10) wherein the bead-like support is streptavidin beads.
(12) The method according to (10) wherein the plate-like support is streptavidin plates or DNA microarray plates.
(13) The method according to any one of (1) to (12) wherein the method is employed in cDNA library preparation, a sequencing reaction, a DNA synthesis reaction, or GSC.
(14) The method according to (13) wherein the DNA synthesis reaction is emulsion PCR or bridge PCR.

### Effect of the Invention

The present invention reduces or suppresses the inhibitory effect of a support upon which a target substance has been immobilized on the reactivity of an enzyme on the target substance by means of a simple, safe, and inexpensive method of simply adding one or more substances selected from the group consisting of saccharides such as trehalose, amino acids, polyhydric alcohols, and derivatives thereof. That is, the present invention promotes DNA synthesis reactions, restriction enzyme reactions, nucleic acid degradation reactions, sequencing reactions, and the like on target DNA that has been immobilized on a support in a simple, safe, and inexpensive manner.

The present invention enhances the reactivity of DNA synthesis in the vicinity of the surface of a support, permitting the subsequent recovery of highly pure DNA, its specific separation, its easy purification, a reduction in operating time, and the like. The present invention can be applied to enzymatic reactions under a variety of conditions involving target substances such as nucleic acids and peptides immobilized on supports. An advantageous effect can be achieved even in cases where it is difficult to maintain the reactivity of an enzyme due to the support. Accordingly, the present invention can be applied not just to cDNA library preparation and the GSC method, but also to large-scale automated sequencing systems such as the Solexa sequencing system and the 454 sequencing system. It can be expected to enhance the performance of such systems.

### Brief Description of the Drawings

[Fig. 1] Fig. 1 shows the results of measurement of the dependency of the change in the quantity of 50 bp DNA fragments on trehalose concentration.
[Fig. 2] Fig. 2 shows the results of measurement of the quantity of double-stranded cDNA synthesized.
[Fig. 3] Fig. 3 shows the results of measurement of the reaction efficiency of various restriction enzymes.
[Fig. 4] Fig. 4 shows the results of measurement of the titer of a cDNA phage library.
[Fig. 5] Fig. 5 shows the results of measurement of the reaction efficiency of restriction enzymes on various support surfaces.
[Fig. 6A] Fig. 6A shows the results of the measurement of various distances from the reaction surfaces of various supports to the position of the DNA cleavage reaction, the measurement of the reaction efficiencies thereof, and a comparison of the support shapes by distance.
[Fig. 6B] Fig. 6B shows the results of the measurement of various distances from the reaction surfaces of various supports to the position of the DNA cleavage reaction, the measurement of the reaction efficiencies thereof, and a comparison of the distances by support shape.
[Fig. 7] Fig. 7 shows the results of the measurement of enzymatic reaction efficiencies under various reaction conditions.
[Fig. 8] Fig. 8 shows the state of a static reaction of restriction enzyme on DNA immobilized on beads after three hours.
[Fig. 9] Fig. 9 shows the results of measurement of the difference in activity of an enzyme in a cleavage reaction on a bead surface and on a plate surface.
[Fig. 10] Fig. 10 shows the results of measurement by a Solexa sequencing system.
[Fig. 11] Fig. 11 shows DNA fragments obtained by PCR amplification of cDNA obtained by reverse transcription of short RNA.
[Fig. 12] Fig. 12 shows the results of a comparison of the number of DNA amplification wells in a 454 sequencing system.
[Fig. 13] Fig. 13 shows the results of a 454 sequencing system.
[Fig. 14] Fig. 14 shows the evaluation results of the number of read sequences and the number of cDNA tags as affected by trehalose in emulsion PCR in a 454 sequencing system.
[Fig. 15] Fig. 15 shows the results of evaluation for different sizes of DNA fragments as affected by trehalose in a uniform liquid phase system.
[Fig. 16] Fig. 16 shows the results of evaluation by electrophoresis as affected by trehalose in a uniform liquid phase system.
[Fig. 17] Fig. 17 shows the results of measurement of enzymatic reaction efficiency in the presence of the various reagents of Example 13 when added in various concentrations.
[Fig. 18] Fig. 18 shows the results of measurement of the 454 sequencing system in Example 14.
[Fig. 19] Fig. 19 shows the results of measurement of the 454 sequencing system in Example 14.
[Fig. 20] Fig. 20 shows the results of measurement of the Solexa sequencing system in Example 15.
[Fig. 21] Fig. 21 shows the results of measurement of the Solexa sequencing system in Example 15.
[Fig. 22] Fig. 22 shows the results of measurement of the Solexa sequencing system in Example 16.
[Fig. 23] Fig. 23 shows the results of measurement of the Solexa sequencing system in Example 16.
[Fig. 24] Fig. 24 shows the results of measurement of the SOLiD sequencing system in Example 17.
[Fig. 25] Fig. 25 shows the results of measurement of the 454 sequencing system in Example 18.
[Fig. 26] Fig. 26 shows a summary of the GSC method employing beads.
[Fig. 27] Fig. 27 shows the synthesis of double-stranded cDNA in a system immobilized on streptavidin beads (Gubler-Hoffman method).
[Fig. 28] Fig. 28 shows restriction enzyme reaction of double-stranded cDNA in a system immobilized on streptavidin beads.
[Fig. 29] Fig. 29 shows the preparation of cDNA phage library.
[Fig. 30] Fig. 30 shows sequences of DNA fragments.
[Fig. 31] Fig. 31 shows restriction enzyme reaction on various reaction surfaces in the presence or absence of D(+)-trehalose.
[Fig. 32] Fig. 32 shows cleavage reaction of DNA fragments with restriction enzyme (PstI) site at different site.
[Fig. 33] Fig. 33 shows restriction enzyme (HindIII) reaction under various reaction conditions.
[Fig. 34] Fig. 34 shows restriction enzyme reaction cleaving the same site with different enzymes.
[Fig. 35] Fig. 35 shows a template amplification reaction under conditions where trehalose was added.
[Fig. 36] Fig. 36 shows the flow of a large-scale 454 sequencing system.
[Fig. 37] Fig. 37 shows emulsion PCR under conditions where trehalose was added.

### [Modes of Carrying Out the Invention]

Modes of carrying out the invention will be described in detail below.

The methods of the present invention comprise a method for reducing or suppressing the inhibitory effect of a support on the reactivity of an enzyme to a target substance that has been immobilized on the support by placing the enzyme in the presence of one or more substances selected from the group consisting of saccharides, amino acids, polyhydric alcohols. The present invention improves the reactivity of an enzyme to a target substance immobilized on a support by placing the enzyme in the presence of one or more substances selected from the group consisting of saccharides, amino acids, polyhydric alcohols relative to when the enzyme is not in the presence of one or more substances selected from the group consisting of saccharides, amino acids, polyhydric alcohols.

The saccharide can be, for example, an oligosaccharide or a monosaccharide. Specific examples of the saccharide are: trehalose, maltose, glucose, sucrose, lactose, xylobiose, agarobiose, cellobiose, levanbiose, quitobiose, 2-β-glucuronosylglucuronic acid, allose, altrose, galactose, gulose, idose, mannose, talose, sorbitol, levulose, xylitol and arabitol. Of these, trehalose, glucose, and sorbitol are desirably employed. The saccharides can be employed singly, or in combinations of two or more.

The amino acid, or a derivative thereof, is selected, for example, from the group consisting of N^{e}-acetyl-β-lysine, alanine, *gamma*-aminobutyric acid, betaine, glycine betaine, N^{a}-carbamoyl-L-glutamine-1-amide, choline, dimethylthetine, ecotine, glutamate, β-glutamine, glycine, octopine, proline, sarcosine, taurine, and trimethylamine N-oxide. The above amino acids can be employed singly or in combinations of two or more. Betaine and sarcosine are preferably employed.

The polyhydric alcohol includes polyhydric alcohols of the above saccharides. Additional examples of polyhydric alcohols are glycerol, ethylene glycol, and polyethylene glycol. Of these, ethylene glycol is desirable. These polyhydric alcohols may be employed singly or in combinations of two or more.

The saccharides, amino acids, polyhydric alcohols, and derivatives thereof may be employed in the form of synthesized products or commercially available products. Their hydrates and anhydrates may also be employed. The quantity of the saccharides, amino acids, polyhydric alcohols, and derivatives thereof that are employed is not specifically limited. The quantity can be suitably adjusted based on the quantity of enzyme, the inhibitory effect of the support on the reactivity of the enzyme, and the like. For example, when employing trehalose or sorbitol alone, the final concentration of trehalose or sorbitol in the solution is 0.1 to 1 M, desirably 0.4 to 0.8 M, and preferably 0.6 M.

The "target substance" that is referred to in the present specification is not specifically limited other than that it be a substance that is targeted by the enzyme employed. Examples are nucleic acids, peptides, saccharides, and fatty acids. Nucleic acids are desirable, and nucleic acids obtained by hybridizing RNA and DNA and single-stranded and double-stranded DNA are preferred.

The "enzyme" that is referred to in the present specification is not specifically limited beyond the conventional meaning of the term. Examples are oxidoreductases (EC 1), transferases (EC 2), hydrolases (EC 3), lyases (EC 4), isomerases (EC 5), and synthases (EC 6). The following enzymes are examples of the above: DNA ligases such as T4 DNA ligase and *E. coli* DNA ligase; RNA ligases such as T4 RNA ligase; polynucleotide kinases such as T4 polynucleotide kinase and polynucleotide kinase; alkaline phosphatases such as alkaline phosphatase (*E*. *coli* C75), alkaline phosphatase (calf intestine), and alkaline phosphatase (shrimp); DNA polymerases such as DNA polymerase I and T4 DNA polymerase; reverse transcriptases such as prime script reverse transcriptase, reverse transcriptase XL, and M-MLV reverse transcriptase; terminal deoxynucleotidyl transferase; RNA polymerases such as SP6 RNA polymerase, and T7 RNA polymerase; poly(A)polymerase; nucleases such as S1 nuclease, mung bean nuclease, micrococcal nuclease, and BAL 31 nuclease; exonucleases such as exonuclease I and exonuclease III; deoxyribonuclease I; DNasel; RNases such as RNaseH and RNaseA; DNA topoisomerase I; DNA methyltransferase; DNA glycosylase; and methylase.

Desirable examples of the above enzymes are as follows. DNA polymerase, a transferase, is desirable, and DNA polymerase I is preferred. RNase, a hydrolase, is desirable, and RNaseH is preferred. DNA ligase, a synthase, is desirable and E. coli DNA ligase is preferred. Transcriptase, a transcription enzyme, is desirable, and M-MLV reverse transcriptase is preferred. However, the enzyme is not limited to these enzymes.

Restriction enzymes are included in the above desirable examples of enzymes. Examples of restriction enzymes are any restriction enzyme that is employed in genetic engineering. Examples are Styl, EcoRI, Mlul, Ncol, DNasel, RNaseI, NdeI, PvulI, PstI, Dral, XhoI, NotI, HindIII, and HincII. However, the enzyme is not limited to these enzymes.

In the method of the present invention, the temperature can be set based on the optimal temperature and deactivation temperature of the enzyme employed. The method of the present invention is normally conducted at a temperature of 1 to 100°C, desirably at a temperature of 5 to 60°C, and preferably at a temperature of 10 to 40°C. Specifically, when synthesizing double-stranded cDNA by the Gubler-Hoffman method using enzymes in the form of *E*. *coli* DNA ligase, DNA polymerase, and RNaseH, the method of the present invention can be conducted at a temperature of 16°C. Further, when conducting a restriction enzyme reaction with enzyme in the form of a restriction enzyme, the method of the present invention can be conducted at a temperature of 37°C.

The "target substance immobilized on a support" and "support on which a target substance is immobilized" that are referred to in the present invention are not specifically limited other than that they be a target substance and a support such that at least a part of the target substance is attached to at least part of the support. Such attachment may be achieved by means of ionic bonds, hydrogen bonds, hydrophobic bonds, covalent bonds, van der Waals bonds, affinity bonds, physical or chemical adsorption, or the like. The method of attachment of the support and the target substance is not specifically limited; for example, the target substance can be attached to the surface of the support, or the target substance can be embedded in the support.

The "support" referred to in the present invention is not specifically limited so long as it permits the attachment of the target substance. The shape thereof can be that of beads, plates, fiber, tubes, containers (a test tube or a vial), or the like. Of these, beads and plates are desirable. The material of the support can be organic or inorganic. Examples are glass, cement, ceramics such as pottery, new ceramics, polyethylene terephthalate, cellulose acetate, polycarbonates of bisphenol A, polystyrenes, polymethyl methacrylate, and other polymers; silicon, activated charcoal, porous glass, porous ceramics, porous silicon, porous activated charcoal, textiles, knitted goods, nonwoven fabric, filter paper, short fiber, membrane filters, and other porous materials; and electrically conductive materials such as gold. Of these, glass and silicon are desirable. The surface of the support can be treated to incorporate functional groups such as amino groups, carboxyl groups, or hydroxy groups; macromolecules such as streptavidin; or the like. Specific examples of such carriers are streptavidin beads, streptavidin plates, and DNA microarray-use plates. The size of the support is not specifically limited. However, a size such that the enzymatic reaction is confirmed to decrease based on the support is desirable. For example, when employing a bead-like support, a diameter of 0.5 micrometer or greater is normally employed, 1 micrometer or greater is desirable, 1.5 micrometers or greater is preferred, 2 micrometers or greater is of greater preference, and 2.5 micrometers or greater is of still greater preference. The upper limit of the diameter of the support is not specifically limited, but a diameter of about 10 to 100 micrometers is employed for bead-like supports. MAGNOTEX-SA from Takara is a specific example of a bead-like support. The diameter of the bead-like support is 2.3 ± 0.3 micrometer.

In the method of the present invention, the distance from the support to the reaction site of the enzyme on the target substance immobilized on the support is, in the case of a target substance in the form of a nucleic acid, for example, 1 to 200 bases from the support, desirably 5 to 150 bases, preferably 5 to 100 bases, more preferably 10 to 80 bases, still more preferably 10 to 50 bases, and optimally, 10 to 20 bases. However, this distance will vary with the target substance and the type of enzyme.

Generally, the closer the support is to the position of the reaction site of the target substance immobilized on the support, the greater the reduction in the reactivity of the enzyme. However, with the method of the present invention, the closer this position becomes, the more the reactivity of the enzyme tends to increase, irrespective of the shape or material of the support. For example, in the method of the present invention, when beads or plates are employed as the support, restriction enzyme is employed as the enzyme, and double-stranded DNA in which the restriction enzyme site is 20 base pairs, 50 base pairs, or 100 base pairs from the support is employed as the target substance, the reactivity of the enzyme tends to increase in the order of double-stranded DNA separated by 20 base pairs, DNA separated by 50 base pairs, and DNA separated by 100 base pairs, irrespective of whether the support is comprised of beads or plates.

When the method of the present invention is applied to the preparation of a cDNA library, high-quality cDNA tends to be recovered with good efficiency. For example, the method of the present invention can be applied to the preparation of a λ phage cDNA library. As a specific example, mRNA is reverse transcribed with biotinylated primer to synthesize a cDNA/mRNA hybrid. The hybrid is immobilized on streptavidin beads or streptavidin plates. Next, the method of the present invention is applied to synthesize double-stranded cDNA. The cDNA that is recovered is ligated to a A phage vector, to prepare a A phage cDNA library. The titer of the A phage cDNA library that is thus prepared can be anticipated to be higher than that when the method of the present invention is not applied.

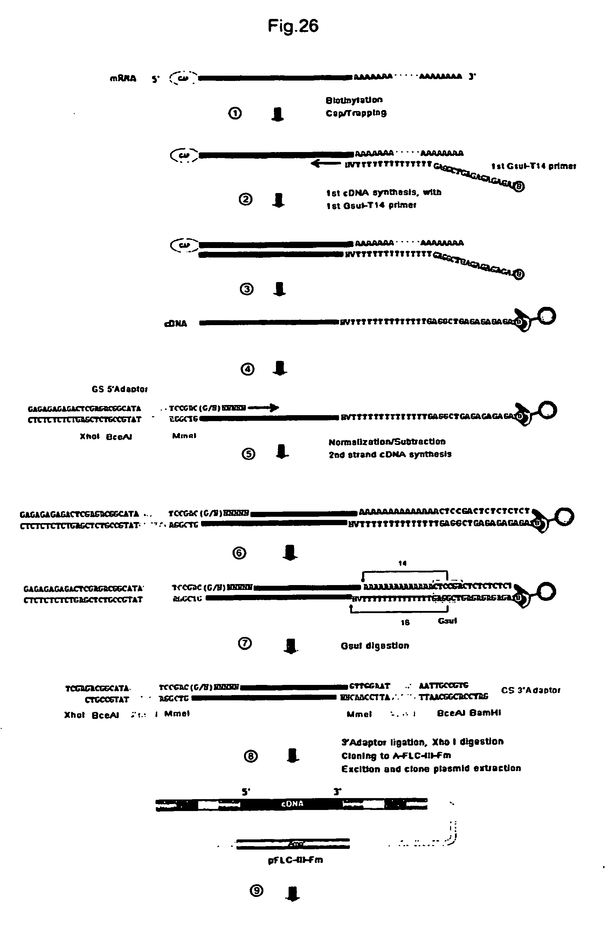

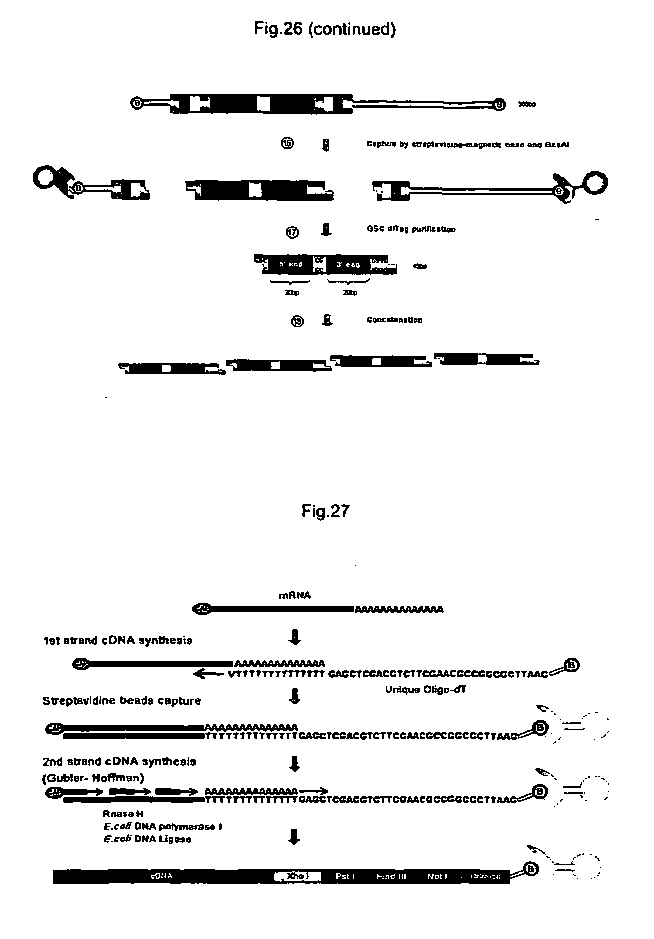

The method of the present invention can, for example, be applied to the GSC method. The GSC method is a method of preparing a cDNA library. When using this method, the 5' end and 3' end of the cDNA can be minimized, and a large amount of cDNA information can be obtained in a single sequencing reaction. A summary of the GSC method employing beads will be described according to Fig. 26.

Single-strand cDNA is synthesized from mRNA using 1st Gsul-T14 primer the 5' end of which has been biotinylated (see 1 to 4 in Fig. 26). GS 5' adaptor is ligated to the synthesized cDNA. Next, a complementary strand of cDNA is synthesized (5 and 6 in Fig. 26). The double-stranded cDNA obtained is processed with Gsul and GS 3' adaptor is ligated to the cDNA. Next, processing is conducted with Xhol to obtain cDNA with adaptors ligated to both ends. The cDNA obtained is adjusted by packaging and the like with phage vector to clone λ-pFLC-III-Fm plasmid (9 in Fig. 26). Following cloning, the plasmid is sequentially processed with Mme1, ligated with GS Fill-in Adaptor, and processed with Mva1269I. Subsequently, self-ligation yields a plasmid construct comprising the 5' end and 3' end of cDNA (10 to 14 in Fig. 26). The plasmid construct obtained is PCR amplified with GS primer R1-Biotin and GS primer F1-Biotin with biotinylated ends. The amplification product obtained is scavenged with streptoavidin beads. Next, treatment with BceAI is conducted to obtain GSC diTags in the form of DNA fragments containing the 5' end and 3' end of the cDNA (15 to 17 in Fig. 26). The GSC diTags can be joined together following purification (18 in Fig. 26). Following this procedure yields GSC diTags with minimized cDNA 5' end and 3' end base sequences. By joining the GSC diTags and sequencing them, a large amount of cDNA information can be obtained in a single sequencing. The above GSC method, by being based on such a procedure, is an extremely useful method permitting the large-scale detection of transcription start sites and transcription terminators.

In the above GSC method, the method of the present invention can be applied to the step of obtaining single-stranded cDNA (1 to 4 in Fig. 26) and the step of obtaining GSC diTags (15 to 17 in Fig. 26). That is, by employing the above single-stranded cDNA and GSC diTags that are target substances in the GSC method as a target substance that has been immobilized on a support, the method of the present invention can be applied to the GSC method. Applying the method of the present invention to the GSC method in this manner improves the recovery rate of the above single-stranded cDNA and GSC diTags. The methods described in the Examples are specific examples of this application.

Further, the method of the present invention can be employed in sequencing reactions and DNA synthesis reactions such as PCR on a support. For example, when employing DNA microarray plates as a support, single-stranded DNA on a support as the target substance, and DNA polymerase as the enzyme, the method of the present invention can be applied to the cluster amplification reaction based on bridge PCR in the Solexa sequencing system. The Solara sequencing system is a sequencing system provided by Illumina, Inc. (see http:www.illuminakk.co.jp/, the contents of the entirety of which are incorporated herein by reference) that permits large-scale sequencing to simultaneously determine in parallel the base sequences of up to several million DNA fragments. Due to extremely high stability and accuracy, it is an effective sequencing system that permits the acquisition of large amounts of sequencing data with good accuracy and provides large-scale DNA analysis with high productivity, good economy, and good accuracy. When the method of the present invention is applied to bridge PCR in the Solexa sequencing system based on the description in the Examples, for example, the cluster amplification reaction, that is, the number of clusters of template DNA that can be amplified, is increased by enhancing the reactivity of the DNA polymerase in DNA synthesis. The intensity of florescent coloration of each base intensifies, resulting in an increased level of sequencing. When applied to the sequencing reaction in a Solexa sequencing system, the enzymatic activity increases in the sequencing reaction, more molecules undergo the elongation reaction, affording improvement in the drop in fluorescence emission intensity accompanying the reaction cycles and increasing the mapping rate of the sequences that have been determined to the genome.

As another example, the method of the present invention can be applied to the sequencing system employed by the 454 sequencer (454 Life Sciences: Roche). More specifically, the method of the present invention can be applied to the emulsion PCR and sequencing reaction, in which PCR is conducted on beads, in the 454 sequencing system.

The '454 Sequencer' (Genome Sequencer FLX System) is a revolutionary method of rapidly decoding and assembling the entire genome sequence. It is a large-scale sequencer that provides powerful processing capability in read length and read numbers. An average length of 200 to 300 bases is read in each read. Since the base pair sequences of 400,000 reads are decoded, the sequence data of more than 100 million base pairs can be analyzed over 7.5 hours in a single run. Further, the individual base pair decoding precision of sequences exceeding 200 base pairs that have been read is 99.5% or greater. In the 454 sequencing system, DNA template is adsorbed onto spherical beads, immobilized, and subjected to enveloped amplification in a water-in-oil emulsion, that is, emulsion PCR. For example, as indicated in the Examples, the application of the present invention to emulsion PCR increases the enzymatic activity in emulsion PCR and markedly increases the sequencing analysis level. The method of the present invention can also be applied to the sequencing reaction following emulsion PCR.

As a separate example, the method of the present invention can be applied to a sequencing system employing a SOLiD sequencer (Applied Biosystems). More specifically, the method of the present invention can be applied to both the emulsion PCR in which PCR is conducted on beads and the sequencing reaction in the SOLiD sequencing system. For example, as indicated in the Examples, applying the present invention to emulsion PCR increases the enzymatic activity in emulsion PCR and markedly increases the level of sequencing analysis. The application of the method of the present invention to the sequencing reaction following emulsion PCR also affords improvement with respect to increased noise.

In addition, the method of the present invention can be applied to sequencing systems employing the next generation of sequencers, such as the Helicos sequencer (Helicos Biosciences Corp.).

The present invention is described more specifically below through Examples. However, the present invention is not limited thereto. Various changes and modifications are possible by persons having ordinary skill in the art. Such changes and modifications are also covered by the present invention.

### [Examples]

### 1. Experimental,materials

D(+)-trehalose in the form of Fluka Biochemika 90208 was employed. D(+) sorbitol in the form of Fluka Biochemika (85529) was employed. D(+) glucose in the form of Wako (041-00595) was employed. And betaine in the form of Wako (023-10862) was employed. Streptavidin beads in the form of MAGNOTEX-SA 9088 from Takara were employed. Streptavidin plates in the form of BioBind Streptavidin coat plates (FN-95029263) from Thermo Science were employed. The following enzymes were employed: M-MLV reverse transcriptase (Promega 90208), RNaseH (NEBM0297S), E. coli DNA polymerasel (NEB0209S), E. coli DNA ligase (NEB M0205S), Xhol (NEB R0146S), PstI (NEB R0140S), HindIII (NEB R0104S), NotI (NEB R0189S), and EcoRI (Fermentas ER0273). MAXplax Packaging Extract MP5120 from Epicentre was employed in phase packaging. The DNA employed is given in Seq. ID Nos. 1-6 of the Sequence Listing.

### 2. DNA fragment cleavage in the presence of D(+)-trehalose

Biotin-labeled 300 bp DNA fragments adsorbed (bound) to streptavidin beads were reacted with the restriction enzyme BceAI and the final concentration of the trehalose that was added to the system was examined as follows.

### DNA fragment cleavage

PCR was conducted with biotin-labeled primers (Seq. ID Nos. 1 and 2) on 300 bp DNA fragments using pFLCIII vector as template. The fragments were cleaved at the type II restriction enzyme site BceAI, releasing 50 bp DNA fragments that were detected. Paramagnetic streptavidin beads were washed with accessory 1x binding buffer, recovered with a magnetic stand to remove the supernatant, and then suspended in the same buffer. Equal volumes of the PCR product-containing solution and 2x binding buffer were mixed, the bead solution was added, and the mixture was gently stirred with top to bottom inversion and rotation for 15 minutes at room temperature to induce adsorption. The magnetic stand was used to recover the sample adsorption beads, which were washed three times with 1x binding buffer. They were then washed once with 1x BceAI buffer. Two reaction solutions (1x BceAI buffer, 1x BSA, restriction enzyme (BceAI) 20 units) were prepared: a trehalose (+) system with a final D(+)-trehalose concentration of 0.6 M, and a trehalose (-) system, to which no trehalose was added. The reaction solutions were added to washed beads, mixed, and reacted for 3 hours at 37°C. The beads were recovered with the magnetic stand and cleaved with restriction enzyme. The supernatant containing the cDNA that had been released was reacted with proteinase K, treated with phenol chloroform, and precipitated from ethanol. The cDNA precipitate thus obtained was dissolved in 0.1x TE (pH 7.5) and subjected to 12% PAGE electrophoresis.

### Results

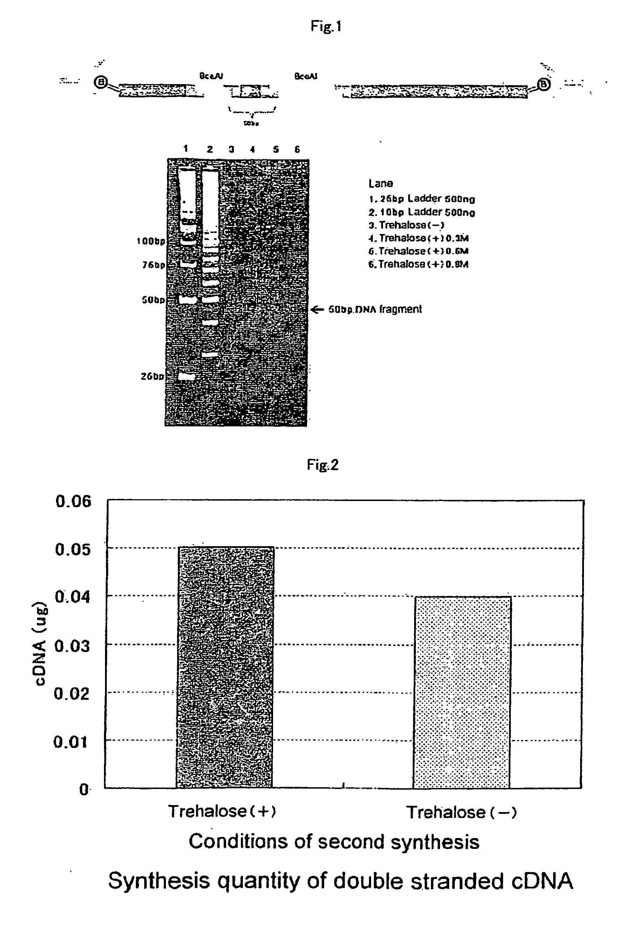

As a result, the yield of 50 bp fragments that were released by cleavage increased when trehalose was added. No change was observed beyond 0.6 M on for 0 M, 0.3 M, 0.6 M, and 0.8 M concentrations (Fig. 1). Accordingly, 0.6 M was set as the optimal concentration in subsequent reactions.

### 3. A comparison of double-stranded cDNA synthesis efficiency

RNA was reverse transcribed with Unique oligo-dT having various restriction enzyme sites. The cDNA/RNA hybrids obtained were used while still adsorbed to the beads to conduct a double-strand synthesis reaction by the Gubler-Hoffman method as set forth below.

### Reverse transcription of RNA (synthesis of single-stranded cDNA)

A 15 ug quantity of 5' end biotin-labeled primer (Seq. ID No. 3) for reverse transcription was employed for 12 ug of total RNA (mouse embryo, 17.5, female). Reverse transcription reaction solution (1x GC buffer (Takara), 0.3 mM dNTP mix, saturated sorbitol/trehalose solution 30 uL, M-MLV 3,000 units) was added α[32P]dGTP was added, and the mixture was reacted for 30 minutes at 42°C, 10 minutes at 50°C, and 10 minutes at 56°C. Following reaction with proteinase K, the mixture was precipitated from CTAB/urea and redissolved in 7 M Gu-HCl. EtOH precipitation was then conducted.

### Adsorption of biotin-labeled cDNA/RNA hybrid onto paramagnetic streptavidin beads

Paramagnetic streptavidin beads were washed with accessory 1x binding buffer, recovered with a magnetic stand to remove the supernatant, and suspended in the same buffer. cDNA/RNA hybrid solution and 2x binding buffer were admixed, the bead solution was added, and the mixture was gently mixed with top to bottom inversion and rotation for 15 minutes at room temperature to induce adsorption. The magnetic stand was used to recover the sample adsorption beads, which were washed three times with 1 x binding buffer.

### Synthesis of double-stranded cDNA (Gubler-Hoffman method)

Streptavidin beads on which was adsorbed cDNA/RNA hybrid were washed once with double-stranded synthesis-use reaction buffer (10x *E. coli* ligase buffer, Invitrogen). Two reaction solutions (1x *E*. *coli* ligase buffer, 0.2 mM dNTP mix, *E. coli* DNA ligase 10 units, *E. coli* DNA polymerase I 40 units, *E. coli* RNaseH 2 units) were prepared: a trehalose (+) system with a final D(+)-trehalose concentration of 0.6M, and a trehalose (-) system to which no trehalose was added. These reaction solutions were mixed with beads that had been washed, α[32P]dGTP was added, and the mixture was reacted for 3 hours at 16°C. The mixture was washed three times with 1x binding buffer and measured with a liquid scintillation counter. The synthesis rate of double-stranded cDNA and the yield were calculated (Fig. 27).

### Results

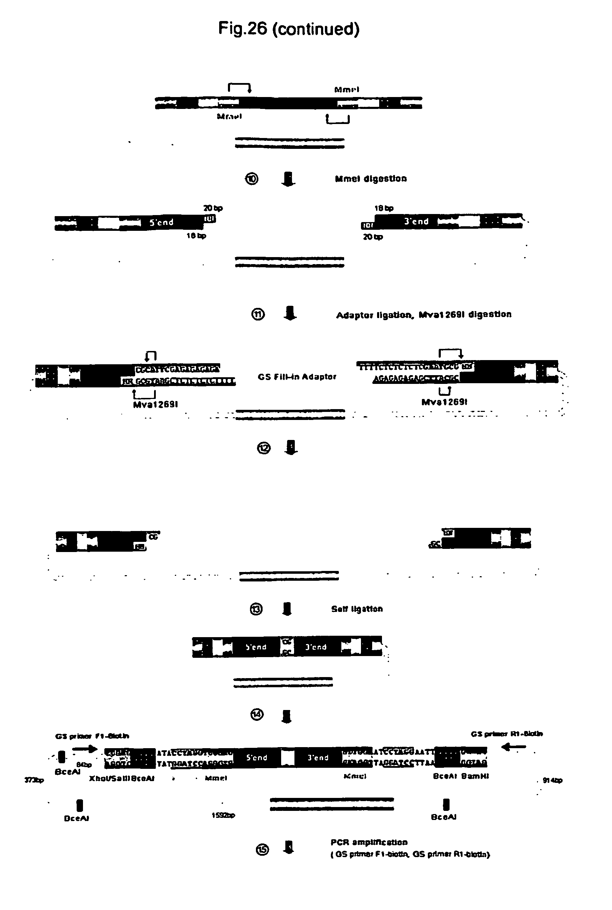

As a result, a comparison of the double-strand synthesis efficiency under the trehalose (+) and trehalose (-) conditions revealed that the synthesis rate under the trehalose (+) conditions exhibited an increase of 1.25-fold over the trehalose (-) conditions (Fig. 2). This indicated that various enzymatic reactions, such as the RNA nicking reaction on the bead surface, the DNA extension reaction, and the DNA ligation reaction were promoted by trehalose.

### 4. Various restriction enzyme reactions on biotin-labeled cDNA immobilized on streptavidin beads

### The restriction enzyme reaction

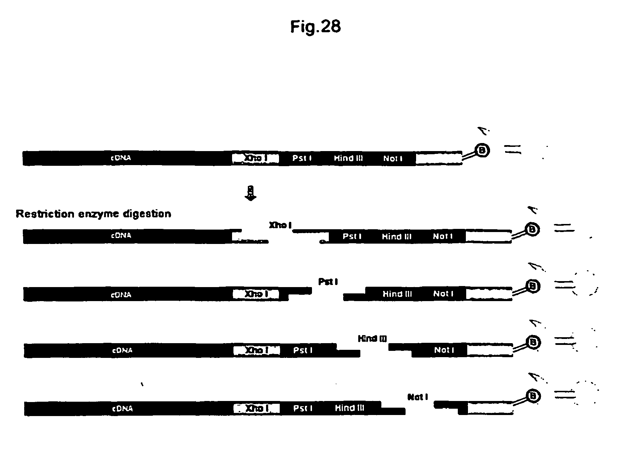

Streptavidin beads on which were adsorbed double-stranded cDNA were washed with various restriction enzyme buffers. The restriction enzymes employed were XhoI, PstI, HindIII, and NotI. Two reaction solutions (various 1x buffers, 1x BSA based on enzyme, restriction enzyme 40 units) were prepared: a trehalose (+) system to which D(+)-trehalose was added to a final concentration of 0.6 M, and a trehalose (-) system, to which no trehalose was added. The reaction solutions were admixed to beads that had been washed and the mixtures were reacted for 3 hours at 37°C. A magnetic stand was used to recover (aggregate) the beads, cleavage was conducted with restriction enzyme, and the supernatant containing the cDNA that had been released was measured with a liquid scintillation counter. The cDNA yield and restriction enzyme efficiency were calculated from the RI count values (Fig. 28).

### Results

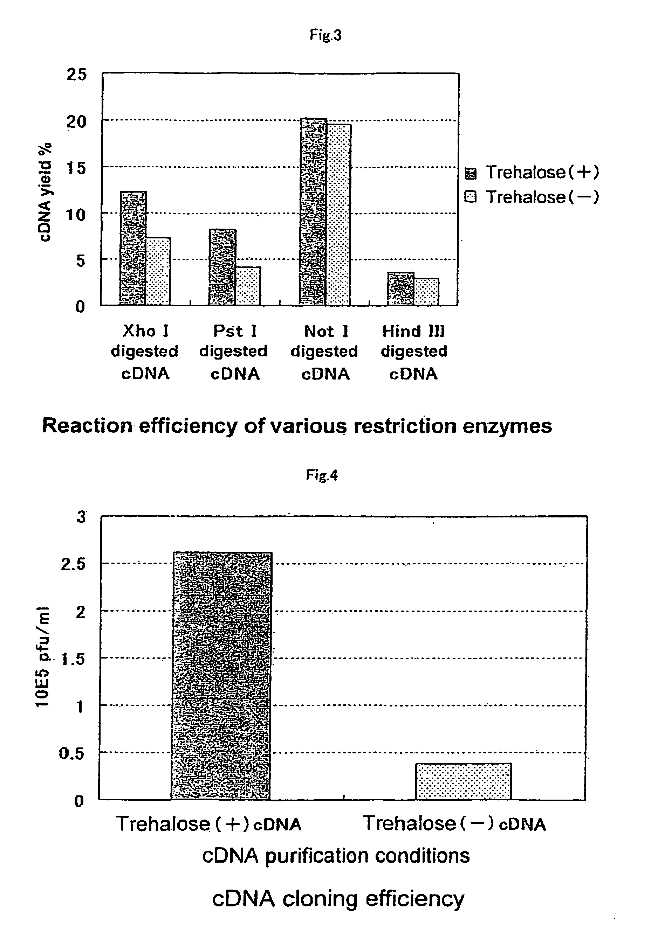

As a result, the various restriction enzyme sites (on the 3' end) on the Unique Oligo-dT employed in the synthesis of single-stranded DNA were cleaved in the double-stranded cDNA that had been synthesized by immobilization on beads. The results of measurement of the quantity of released cDNA that was recovered are given in Fig. 3. In all cases, a higher quantity was recovered from the trehalose (+) system.

### 5. Evaluation of the effect of trehalose on the preparation of a cDNA phase library Preparation of a λ phage cDNA library

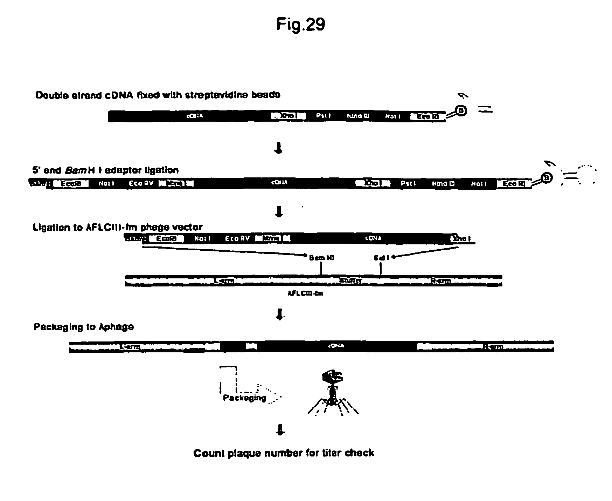

Double-stranded cDNA/RNA hybrid samples that had been immobilized on streptoavidin beads and synthesized under trehalose (+) and trehalose (-) conditions were employed to prepare a λ phage cDNA library. Following synthesis of double-stranded cDNA, the beads were washed with blunting buffer (T4 DNA polymerase buffer). Blunting reaction solution (1x T4 DNA polymerase buffer, 0.6 M trehalose, 0.1 mM dNTP mix, T4 DNA polymerase 5 units) was added and the mixture was incubated for 20 minutes at 12°C. Following the reaction, the beads were washed three times with 0.1 x TE, and then washed one more time with 1x ligation buffer. Ligation reaction solution (1x T4 DNA ligation buffer, 0.6 M trehalose, 5' end BamHI overhang adapter 2 ug, T4 DNA ligase 800 units) was added and the mixture was reacted overnight at 16°C. The beads were washed three times with 0.1x TE and then washed once with 1x Xhol. Restriction enzyme Xhol reaction solution (1x Xhol buffer, 0.6 M trehalose, Xhol 40 units) was added and the mixture was incubated for three hours at 37°C. The beads were recovered with a magnetic stand and the supernatant containing the cDNA that had been released by Xhol cutting was separated. A proteinase K reaction was conducted, treatment with phenol chloroform was carried out, and precipitation by ethanol was conducted. The cDNA precipitate obtained was dissolved in 0.1 x TE (pH 7.5). Ligation was conducted at the molar concentration ratio (cDNA:λ phage vector = 1:1). A reaction with ligation reaction solution (cDNA, λ phage vector, T4 DNA ligase 200 units) was conducted overnight at 16°C. λphage packaging solution (Epicentre) was added and the mixture was left standing for 1 hour and 45 minutes at room temperature. SM buffer and 2 to 3 drops of chloroform were added and the mixture was stored at 4°C. λ phage host bacterium C600 had been inoculated onto 30 mL of LBMM liquid medium (no antibiotic, 10 mM MgSO₄, 0.2% maltose) and cultured for 14 hours at 37°C the previous day were now collected by centrifugal separation and re-suspended in 10 mL of 20 mM MgSO₄. The phage sample solution was diluted 100-fold. A 10 uL quantity and a 100 uL quantity were admixed to the C600/MgSO₄ suspensions to infect them with phage. The infected solutions were added to M-soft agar, quickly vortexed, and spread evenly across the surface of LB agar medium (no antibiotic) by pouring. They were then cultured for 14 to 16 hours at 37°C. The plaques that appeared were counted and the titer was calculated (Fig. 29).

### Results of preparation of cDNA phage library

Double-stranded cDNA/RNA hybrid samples that had been synthesized under trehalose (+) and trehalose (-) conditions and immobilized on beads were employed to prepare a A phage cDNA library. Packaging in A phage was conducted and the titer of the phage library was calculated from the number of plaques that formed. The samples prepared under trehalose (+) conditions exhibited clearly better results (Fig. 4). The structure of the cDNA that had been purified with the addition of trehalose not only promoted the activity of various reactions, but was also of high accuracy, producing a considerable difference in the ultimate vector ligation reaction system.

In the various enzymatic reaction solutions, in which a final D(+)-trehalose concentration of 0.6 M was added to biotin-labeled DNA adsorbed to streptavidin beads, the addition of trehalose diffused the streptavidin beads that had collected at the bottom up to that point in the solution, even in extended reactions, thereby maintaining better uniformity in the reaction solution. Thus, with no nonuniformity in enzymatic activity, the various enzymatic reactions were promoted and the quantity of target DNA recovered increased.

To examine the relation between the shapes of the reaction surface, the cleavage sites, and the reagents added to a DNA fragment cleavage reaction conducted in the presence of trehalose, newly designed DNA fragment reactions were conducted under various conditions as set forth below.

### 6. Evaluation of the shapes of the reaction surface of DNA fragments in the presence of trehalose

Restriction enzyme reactions on biotin-labeled DNA fragments were conducted on support surfaces of various shapes. In this process, systems were established with the presence or absence of trehalose as a reaction condition. How the enzymatic activity associated with differences in the amount of released DNA fragments recovered related to the shape of the reaction surface and the effect of trehalose were examined as set forth below.

### PstI cleavage reaction in solution

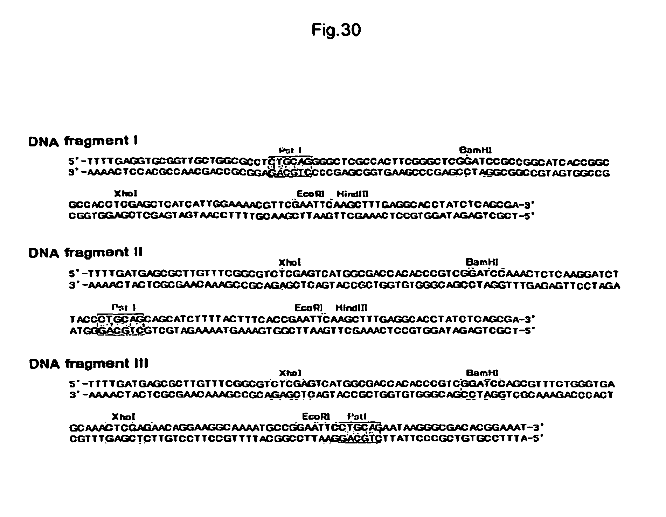

Biotin-labeled DNA fragment III (Seq. ID No. 6, Fig. 30) was cleaved at the restriction enzyme PstI site in solution and the 104 bp DNA fragment that was released was examined. Two forms of reaction solution (DNA fragment III 500 ng, 1 x PSTI buffer, 1x BSA, restriction enzyme 40 units) were prepared: a total of 50 uL trehalose (+) reaction solution to which D(+)-trehalose was added to a final concentration of 0.6 M, and a total of 50 uL trehalose (-) reaction solution, to which no trehalose was added. The reaction solutions were reacted for 3 hours at 37°C, a proteinase K reaction was conducted, phenol chloroform treatment was carried out, and precipitation from ethanol was conducted. The precipitate of the DNA fragment obtained was dissolved in 1x TE (pH 7.5) and analyzed with Bioanalyzer DNA 1000 assay (Agilent Technologies).

### The PstI cleavage reaction on a spherical bead surface

Biotin-labeled DNA fragment III (Seq. ID No. 6, Fig. 30) was cleaved at the restriction enzyme Pstl site on the surface of streptavidin beads and the 104 bp DNA fragment released was examined. DNA fragment III (500 ng) and 2x binding buffer were admixed in equal volume quantities, a solution of beads suspended in 1x binding buffer was added, and the mixture was gently stirred with top to bottom inversion and rotation for 15 minutes at room temperature to induce adsorption. The beads onto which the sample had adsorbed were recovered with a magnetic stand, washed three times with 1 x binding buffer, and washed once with 1 x PstI buffer. Two types of reaction solution (1x PstI buffer, 1 x BSA restriction enzyme 40 units) were prepared: a trehalose (+) system to which D(+)-trehalose was added to a final concentration of 0.6 M, and a trehalose (-) system to which no trehalose was added. Each of the reaction solutions was admixed to beads that had been washed and the mixtures were reacted for three hours at 37°C. The beads were recovered with the magnetic stand and cleaved with restriction enzyme. The supernatant containing the DNA fragments that had been released was reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### The PstI cleavage reaction on a horizontal plate surface

Biotin-labeled DNA fragment III (Seq. ID No. 6, Fig. 30) was cleaved at the restriction enzyme PstI site on a horizontal streptavidin coated plate surface and the 104 bp DNA fragment released was examined. To the DNA fragment III (500 ng) was admixed an equal volume of 2x binding buffer. The mixture was added to the wells of a plate that had been washed with 1x binding buffer and stirred and adsorbed while being gently vibrated for 15 minutes at room temperature. The supernatant was removed, washed three times with 1x binding buffer, and washed once with 1 x PstI buffer. Two types of reaction solution (1 x PstI buffer, 1x BSA restriction enzyme 40 units) were prepared: a trehalose (+) system to which D(+)-trehalose was added to a final concentration of 0.6 M, and a trehalose (-) system to which no trehalose was added. Each of the reaction solutions was added to wells that had been washed, and then reacted for three hours at 37°C. Cleavage was conducted with restriction enzyme. The supernatant containing the DNA fragments that had been released was recovered, reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### The results of comparison of enzymatic activity on various reaction surfaces

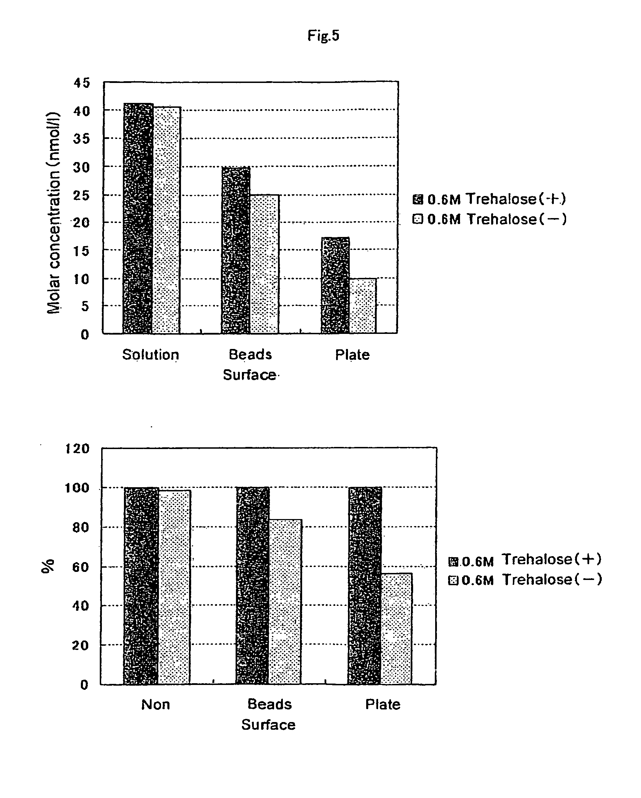

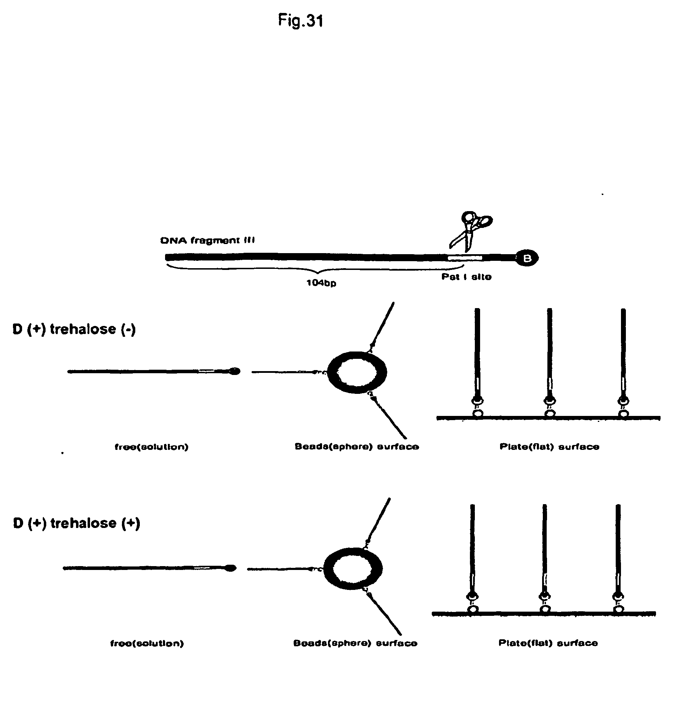

Enzymatic reactions were conducted (Fig. 31) for the three reaction surfaces of a spherical surface in the form of streptavidin beads, a horizontal surface in the form of streptavidin-coated plates, and a free system in the form of normal reaction solution alone. The results are given in Fig. 5. In the normal reaction system, three hours of restriction enzyme reaction with and without the addition of trehalose produced the same yield. In the cleavage reaction of DNA immobilized by adsorption to beads, the addition of trehalose produced a higher yield. The same experiment was conducted with a horizontal surface system in the form of plates, and the addition of trehalose produced a higher yield. These results suggest that enzymatic reactions conducted in ordinary reaction solutions proceed regardless of whether trehalose is added, but in reactions conducted on the surface of a support, the addition of trehalose inhibits the deactivation of enzymatic activity, promoting the reactions.

### 7. Evaluation of the cleavage position of DNA fragments in the presence of trehalose

A comparison was made as set forth below to indicate whether or not there were differences in enzymatic activity based on the distance from the enzyme reaction surface to the DNA recognition site.

### PstI cleavage reactions with different distances from the spherical bead surface to the DNA cleavage reaction site

Three biotin-labeled DNA fragments I, II, and III (Seq. ID Nos. 4 to 6, Fig. 30) were cleaved at their respective restriction enzyme PstI site and the size of the DNA fragments released was examined. Paramagnetic streptavidin beads were washed with the accessory 1x binding buffer and recovered with a magnetic stand to remove the supernatant. They were then suspended in identical buffer. To each of the three types of biotin-labeled DNA fragments (500 ng) was admixed an equal volume of 2x binding buffer, the bead solution was added, and the mixture was stirred gently for 15 minutes with top to bottom inversion and rotation to induce adsorption. The beads to which the sample had adsorbed were recovered with the magnetic stand, washed three times with 1x binding buffer, and washed one more time with 1x Pstl buffer. Two types of reaction solution (1x PstI buffer, 1 x BSA restriction enzyme 40 units) were prepared: a trehalose (+) system to which D(+)-trehalose was added to a final concentration of 0.6 M, and a trehalose (-) system to which no trehalose was added. Each of the reaction solutions was admixed to beads that had been washed, and the mixtures were reacted for three hours at 37°C. The beads were recovered with the magnetic stand and cleaved with restriction enzyme. The supernatant containing the DNA fragments of various sizes that had been released was reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### PstI cleavage reactions with different distances from the horizontal plate surface to the DNA cleavage reaction site

Three biotin-labeled DNA fragments I, II, and III (Seq. ID Nos. 4 to 6, Fig. 30) were cleaved at their respective restriction enzyme PstI site and the size of the DNA fragments released was examined. Three types of biotin-labeled DNA fragments (500 ng) to which equal volumes of 2x binding buffer had been admixed were added to the wells of plates, which had been washed with 1x binding buffer, and stirred while being gently vibrated for 15 minutes at room temperature to induce adsorption. The supernatants were removed, washed three times with 1x binding buffer, and washed once with 1 x Pstl buffer. Two types of reaction solution (1x PstI buffer, 1x BSA restriction enzyme 40 units) were prepared for each: a trehalose (+) system to which D(+)-trehalose was added to a final concentration of 0.6 M, and a trehalose (-) system to which no trehalose was added. Each of the reaction solutions was added to wells that had been washed, and the mixtures were reacted for three hours at 37°C. Cleavage was conducted with restriction enzyme. The supernatant containing the DNA fragments that had been released was recovered, reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### The distance from the enzyme reaction surface to the DNA cleavage reaction site and the effect of trehalose

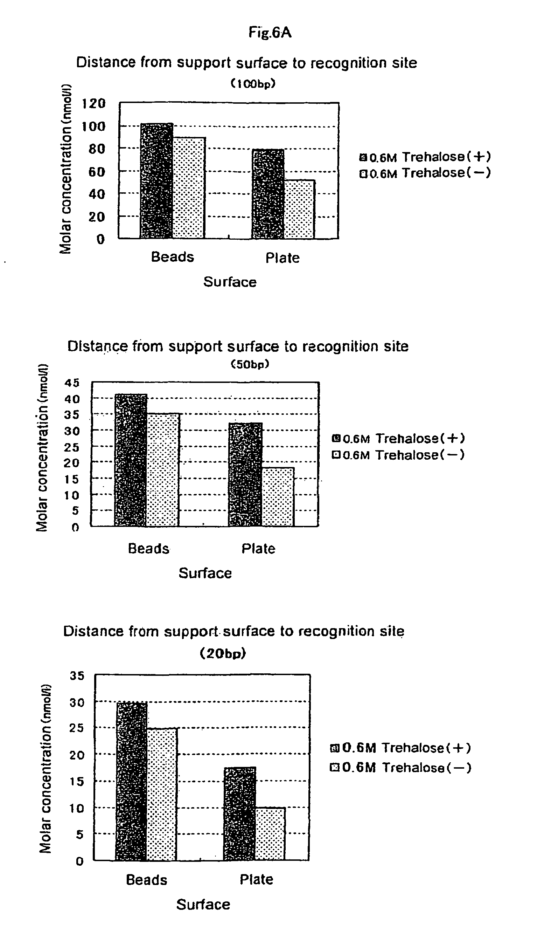

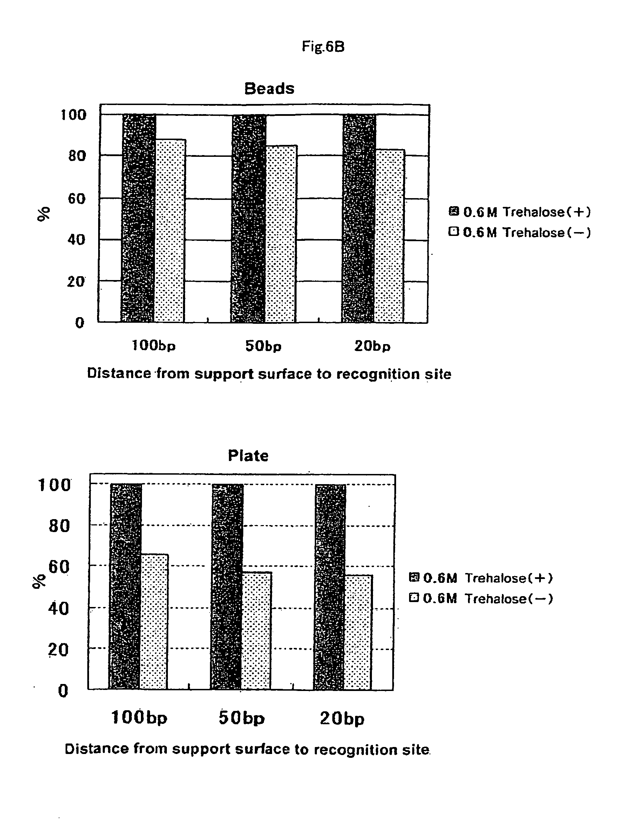

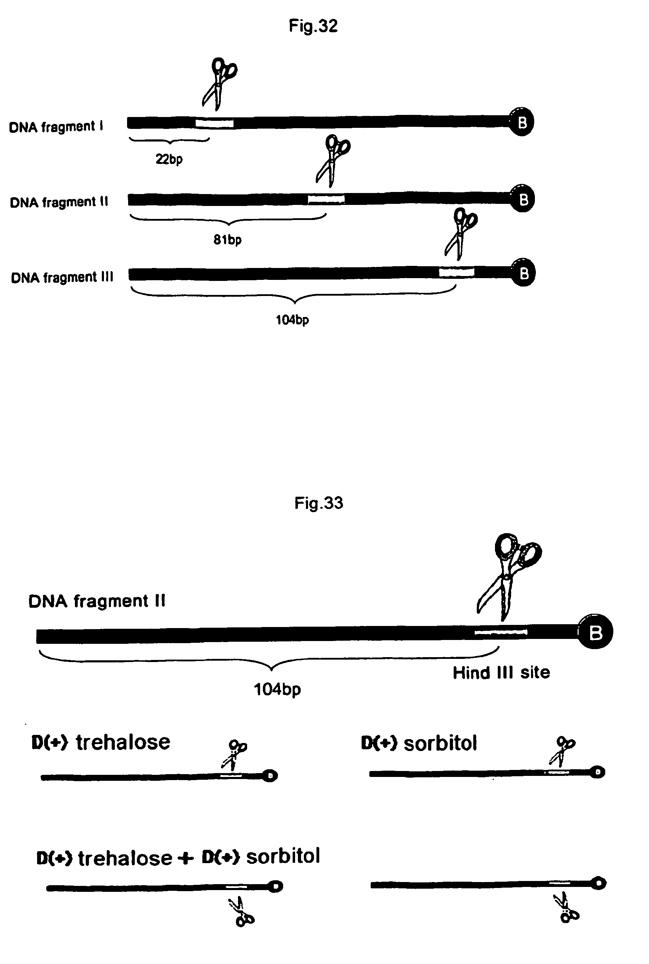

Three stages of cleavage sites were established in order of proximity from the DNA adsorption surface and the cleavage reaction efficiency of each was examined under conditions where trehalose was present and absent. The distances from the surface to the cleavage site were set at 20 bp, 50 bp, and 100 bp, and the yields of the three corresponding DNA fragments of 104 bp, 81 bp, and 22 bp that were released by cleavage were compared and examined (Fig. 32). As a result, the activity of the enzymatic reaction diminished and the yield of DNA decreased with proximity to the surface. However, the addition of trehalose resulted in an increase. The difference became pronounced with increased proximity to the reaction surface (Fig. 6A). The reaction surface shape exhibited the same tendency for both spheres (beads) and flat surfaces (plates) (Fig. 6B).

### 8. Evaluation of the effect of the addition of reagents on the DNA fragment in the presence of trehalose

The effect of the presence of substances other than trehalose on enzymatic activity was examined for enzymatic reactions on the surface of a support. Sorbitol was employed as the added reagent. Trehalose was also added and a sorbitol + trehalose reaction system was examined (Fig. 33).

### DNA fragment HindIII cleavage reactions on spherical bead surfaces under conditions where other substances including D(+)-trehalose were present

HindIII cleavage reactions were conducted on biotin-labeled DNA fragment II (Seq. ID No. 5, Fig. 30) on the surface of beads by adding trehalose, glucose, sorbitol, and betaine, and the 104 bp DNA fragment released was detected. Paramagnetic streptavidin beads were washed with accessory 1x binding buffer, recovered with a magnetic stand to remove the supernatant, and suspended in the same buffer. To the DNA fragment II (500 ng) was admixed an equal volume of 2x binding buffer, the bead solution was added, and the mixture was gently stirred with top to bottom inversion and rotation for 15 minutes at room temperature to induce adsorption. The magnetic stand was used to recover the sample adsorption beads, which were washed three times with 1 x binding buffer and once again with 1x HindIII buffer. Five reaction solutions (1x HindIII buffer, restriction enzyme 40 units) were prepared: a trehalose (+) system with a final D(+)-trehalose concentration of 0.6M, a trehalose (-) system without trehalose, a D(+)-glucose system with a final concentration of 0.6 M, a sorbitol system with a final concentration of 0.6 M, and a betaine system with a final concentration of 2 M. Three combination systems were also prepared with final concentrations of 0.6 M trehalose + 2 M betaine, 0.6 M trehalose + 0.6 M glucose, and 0.6 M trehalose + 0.6 M sorbitol. Each of the reaction solutions was added to beads that had been washed, and the the mixtures were reacted for three hours at 37°C. The beads were recovered with the magnetic stand and cleavage was conducted with restriction enzyme. The supernatant containing the DNA of various sizes that was released was reacted with proteinase K, processed with phenol chloroform, and precipitated from ethanol. The precipitate of DNA fragments thus obtained was dissolved in 1x TE (pH 7.5) and analyzed with Bioanalyzer DNA 1000 assay (Agilent Technologies).

### DNA fragment HindIII cleavage reactions on horizontal plate surfaces under conditions where other substances were present with D(+)-trehalose

Trehalose and sorbitol were added and a HindIII cleavage reaction was conducted on a horizontal plate surface on biotin-labeled DNA fragment II (Seq. ID No. 5, Fig. 30) and the 104 bp DNA fragments that were released were detected. To the wells of a plate that had been washed with 1x binding buffer were added an equal volume mixture of 2x binding buffer and DNA fragment II (500 ng) and the mixture was stirred and adsorbed for 15 minutes at room temperature with gentle vibration. The supernatant was removed, washed three times with 1x binding buffer, and washed once again with 1x HindIII buffer. Reaction solution systems (1x HindIII buffer, restriction enzyme 40 units) were prepared: a trehalose (+) system with a final D(+)-trehalose concentration of 0.6 M, a trehalose (-) system without trehalose, and a sorbitol system with a final concentration 0.6 M. Each of the reaction solutions was added to wells that had been washed, reacted for three hours at 37°C, and cleaved with restriction enzyme. The supernatant containing the DNA fragments released was recovered, reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### Comparative results of enzymatic activity when substances other than trehalose were added

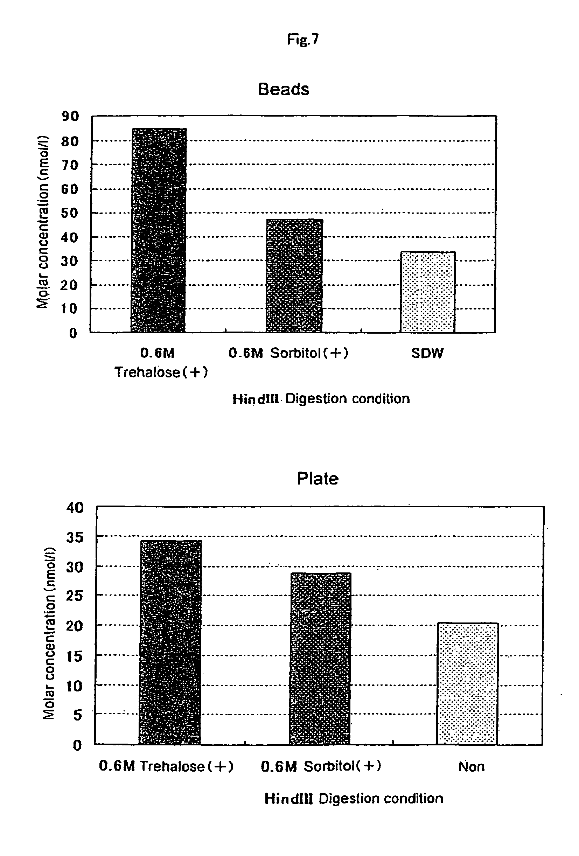

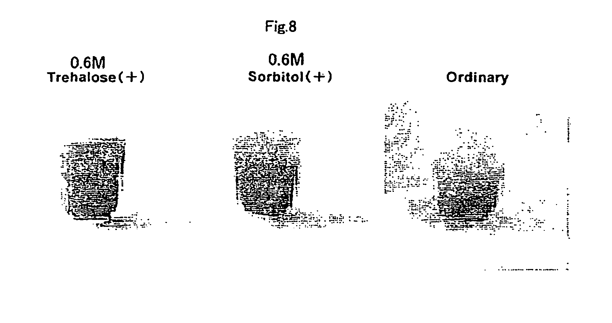

Fig. 7 shows the yields of DNA recovered when cleavage reactions were conducted with restriction enzyme on spheres (beads) and on a horizontal (plate) surface in the presence of various substances. Fig. 8 shows the bead reaction solution following three hours of restriction enzyme reaction for reference. In the same manner as when trehalose was added, the uniformity in the solution of beads was maintained when sorbitol was added. This was attributed to characteristics such as the viscosity and diffusing effect of trehalose or sorbitol. Although not of the same degree as when trehalose was added, high enzymatic activity was exhibited on all reaction surfaces when sorbitol was added.

### 9. Evaluation of the effect of restriction enzyme on DNA fragments in the presence of trehalose

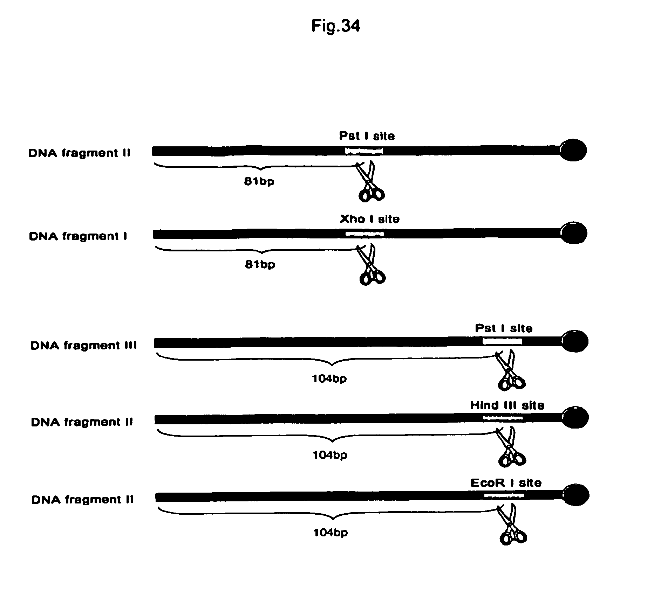

Differences in activity based on the type of enzyme in reactions under conditions where trehalose was present and absent and for various reactions surface shapes were examined. Xhol, Pst!, HindIII, and EcoRI were employed as restriction enzymes to conduct reactions cleaving the same site on a DNA fragment with different enzymes (Fig. 34).

### DNA cleavage reactions by various restriction enzymes on spherical bead surfaces

Cleavage reactions by various restriction enzymes were conducted on biotin-labeled DNA fragments I, II, and III (Seq. ID Nos. 4 to 6, Fig. 30) on bead surfaces. Cleavage reactions at the Xhol and PstI sites (a distance of 50 bp from the bead surface) of DNA fragments I and II resulted in the detection of the release of 80 bp DNA fragments. Cleavage reactions at the EcoRI site of DNA fragment I, the HindIII site of DNA fragment II, and the Pstl site of DNA fragment III (all at distances of 20 bp from the bead surface) resulted in the detection of the release of 104 bp DNA fragments. Paramagnetic streptavidin beads were washed with accessory 1x binding buffer, recovered with a magnetic stand to remove the supernatant, and suspended in the same buffer. To each of DNA fragments I, II, and III (500 nm) was admixed an equal volume of 2x binding buffer, the bead solutions were added, and the mixtures were gently stirred and adsorbed for 15 minutes at room temperature with top to bottom inversion and rotation. The beads onto which the sample had adsorbed were recovered with the magnetic stand and washed three times with 1 x binding buffer. Each DNA fragment was then washed once with 1x Xhol buffer, 1x PstI buffer, 1x HindIII buffer, and 1 xEcoRI buffer. A trehalose (+) system with a final D(+)-trehalose concentration of 0.6 M and a trehalose (-) system to which no trehalose was added were prepared for each of the (1 x Xhol buffer, 1 x BSA, restriction enzyme 40 units), (1x PstI buffer, 1 x BSA, restriction enzyme 40 units), (1x HindIII buffer, restriction enzyme 40 units), and (1x EcoRI buffer, restriction enzyme 40 units) reaction solutions. The various reaction solutions were added to beads that had been washed, and the mixtures were reacted for three hours at 37°C. The beads were recovered with a magnetic stand and cleaved with restriction enzyme. The supernatant containing the DNA of various sizes that had been released was reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### DNA fragment cleavage reactions by different restriction enzymes on a horizontal plate surface

Biotin-labeled DNA fragments II and III (Seq. ID Nos. 5 and 6, Fig. 30) were subjected to cleavage reactions by different restriction enzymes on horizontal streptavidin-coated plate surfaces. Cleavage reactions at the HindIII site of DNA fragment II and the PstI site of DNA fragment III (both at a distance of 20 bp from the plate surface) resulted in the detection of the release of 104 bp DNA fragments. Each of DNA fragments II and III (500 ng) mixed with equal volumes of 2x binding buffer were added to the wells of plates that had been washed with 1x binding buffer and stirred and adsorbed for 15 minutes at room temperature with gentle vibration. The supernatant was removed and washed three times with 1x binding buffer. Each of the DNA fragments was then washed one more time with 1 x HindIII buffer and 1 x PstI buffer. A trehalose (+) system with a final D(+)-trehalose concentration of 0.6 M and a trehalose (-) system to which no trehalose was added were prepared for each of the (1x HindIII buffer, restriction enzyme 40 units) and (1x PstI buffer, 1 x BSA, restriction enzyme 40 units) reaction solutions. The various reaction solutions were added to wells, which had been washed, and reacted for three hours at 37°C. The beads were cleaved with restriction enzyme. The supernatant containing the DNA fragments that had been released was recovered, reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies).

### Comparative results of the enzymatic activity on a reaction surface of different enzymes

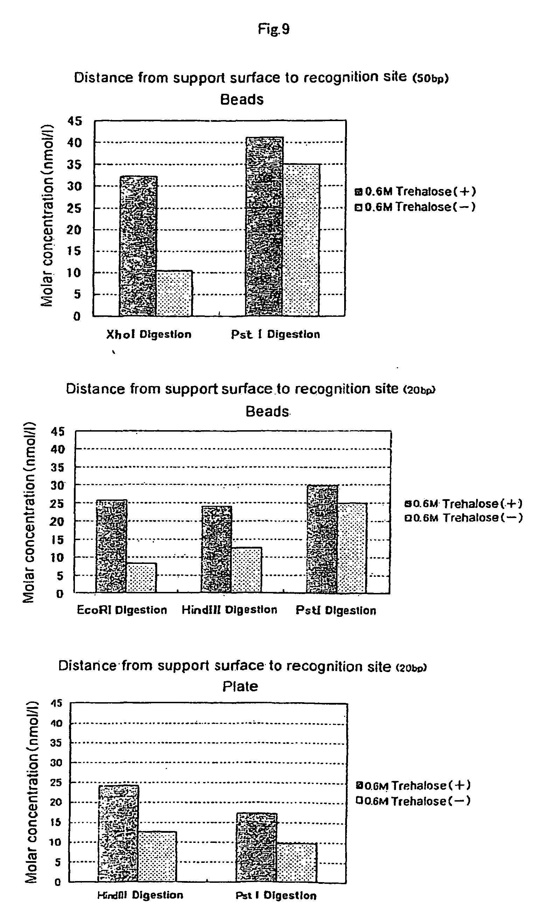

The above results are given in Fig. 9. Differences in activity by enzyme were observed in cleavage reactions on spherical (bead) and horizontal (plate) surfaces. All cases exhibited increased activity with the addition of trehalose.

The effect of trehalose on enzymatic reactions on the surfaces of supports was thought to relate to the promotion of the reaction, or maintenance or enhancement of enzymatic activity, regardless of whether the surface shape was spherical or horizontal. In terms of the relation between enzymatic activity and the distance from the surface of the support to the reaction recognition site of the DNA fragment serving as the enzyme substrate, the activity decreased with proximity to the surface. However, the addition of trehalose enhanced the activity. The three dimensional structure of the enzyme, the specificity of the recognition site, and the like were also thought to be mutually related to trehalose due to this difference in the activity of the enzyme in proximity to the surface. When taken in combination, these results seem to indicate that the role of trehalose in the enzymatic reaction of support-immobilized DNA is not just that of inhibiting the aggregation of a substance such as beads in the reaction solution, but may also involve some direct effect on the enzyme in the surface reaction, the DNA serving as substrate, or a combination of the two.

### 10. Evaluation of the effect of trehalose on DNA sequencing system on DNA chips

The Solexa sequencing system incorporates the steps of adsorbing, immobilizing, and amplifying a DNA template on a flat chip. An examination was conducted to see how the sequence analysis level would change due to the addition of trehalose in the template amplification reaction of the Solexa sequencing system.

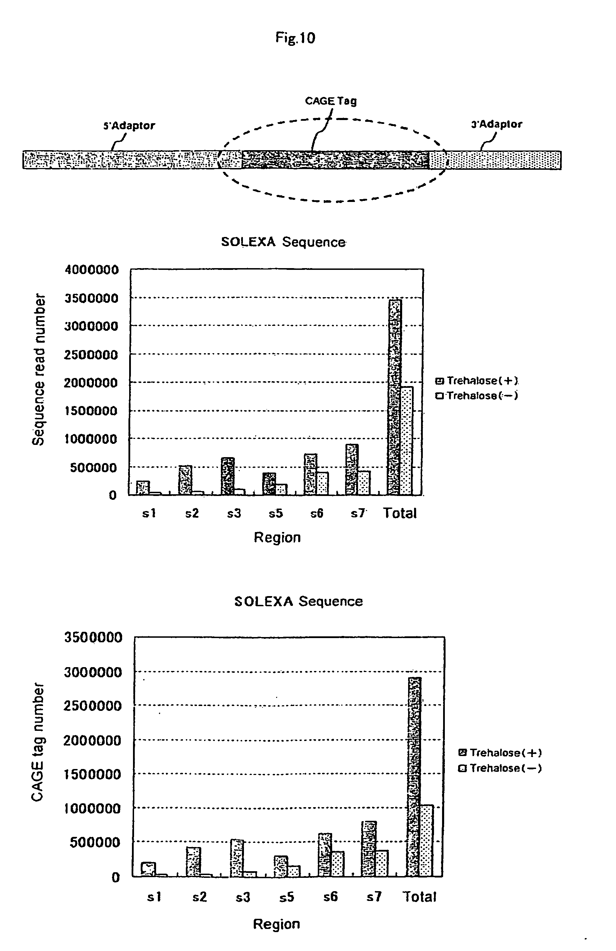

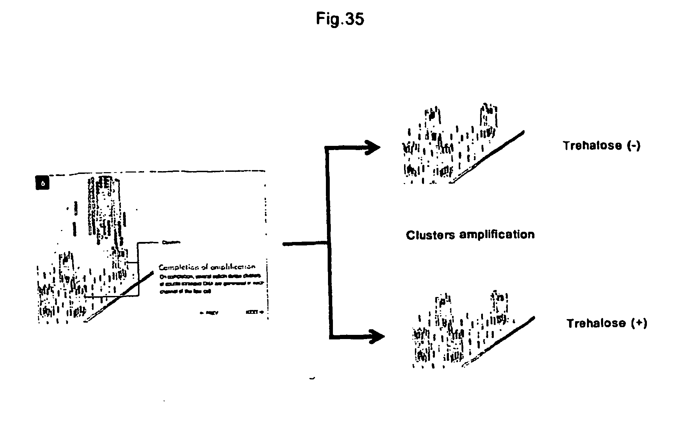

In the process of the Solexa sequencing system, attention was focused on the cluster amplification reaction conducted on a DNA-immobilized chip. In this reaction, DNA immobilized on the chip was amplified with DNA polymerase to obtain identical fragments referred to as a cluster (Fig. 35). Amplification was conducted by adding trehalose to the reaction solution to a final concentration of 0.6 M, and a comparison was made with the results obtained when no trehalose was added (Fig. 10). The sample employed here was a 27 bp DNA fragment that had been prepared by the CAGE method (obtained by cleaving just 27 bp off of the 5' end of cDNA). The results obtained by the subsequent sequencing reaction are given in Table 1.

**[Table 1]**

| **Trehalose(+) cluster amplification** | | |
|---|---|---|
| region | num of sequence read | num of CAGE tag |
| s1 | 252820 | 196840 |
| s2 | 523387 | 421412 |
| s3 | 648786 | 540268 |
| s5 | 392534 | 317440 |
| s6 | 728614 | 627739 |
| s7 | 904101 | 798438 |
| total | 3450242 | **2902137** |
| | | |

| **Trehalose(-) cluster amplification** | | |
|---|---|---|
| region | num of sequence read | num of CAGE tag |
| s1 | 44213 | 28707 |
| s2 | 51227 | 33205 |
| s3 | 104564 | 80463 |
| s5 | 185169 | 154700 |
| s6 | 412311 | 367492 |
| s7 | 422414 | 374732 |
| total | 1919195 | **1039299** |

When the cluster amplification reaction was conducted with the addition of trehalose, the length of the sequence that was determined increased. Among the DNA fragments that were analyzed, more regions prepared by the CAGE method (the CAGE tag) were contained (Fig. 10). In the process of reading the base sequence after the cluster amplification reaction, the fluorescence intensity of each base in the DNA sample that was processed under conditions where trehalose was added was strongly displayed (data not shown). Based on these results, the fact that trehalose affected the efficiency of the enzymatic reaction on a horizontal surface (this time in a DNA extension reaction) indicated that it had effective properties relating to enzymatic activity.

### 11. Evaluation of the effect of trehalose in a DNA sequencing system accompanied by emulsion PCR

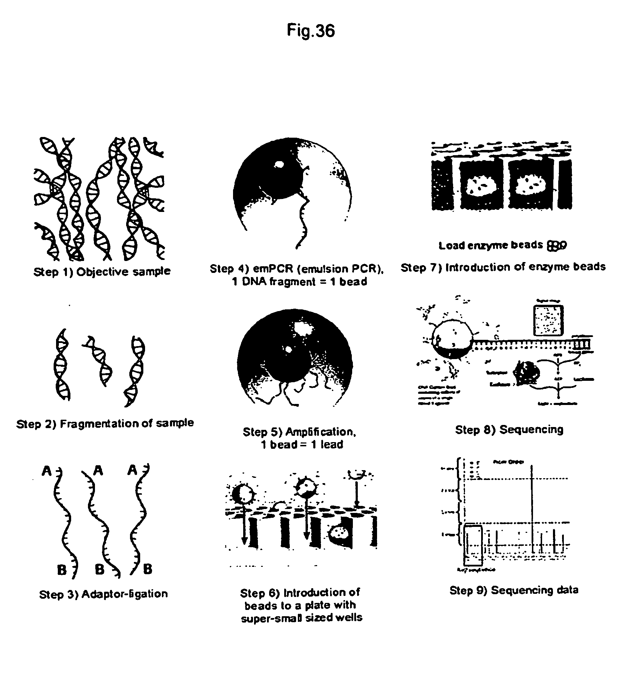

In the 454 sequencing system (Fig. 36), a DNA template was adsorbed onto spherical beads, immobilized, and subjected to enveloped amplification in a water-in-oil emulsion (emulsion PCR). Accordingly, the change in the sequencing analysis level when trehalose was added in emulsion PCR was examined.

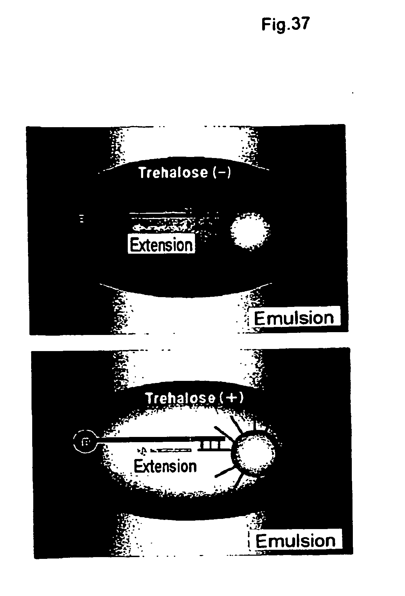

In emulsion PCR, DNA is bonded by complementarily interaction with oligoprimer immobilized on beads and enveloped in a water-in-oil emulsion to form microreactors each comprised of a single bead and a single DNA fragment. An amplification reaction based on the DNA polymerase results in the amplification of each DNA fragment into several million copies per bead. Trehalose was added to a final concentration of 0.6 M to the reaction solution and the amplification reaction was conducted to examine the effect on the sequence analysis results (Fig. 37).

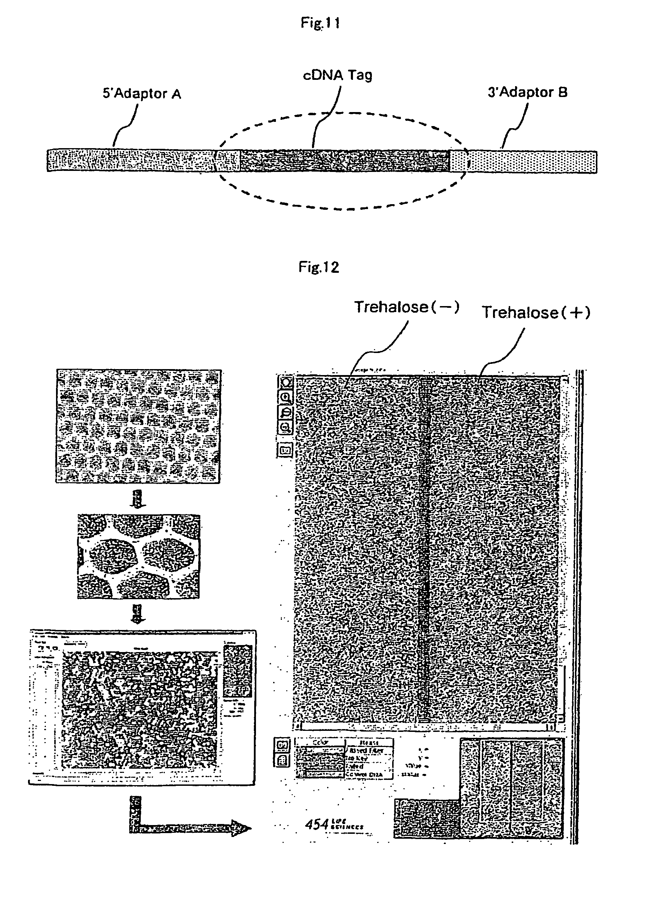

The samples employed were DNA fragments that had been obtained by PCR amplification of cDNA obtained by reverse transcription of short RNA (Fig. 11). The results obtained by this reaction are shown in Figs. 12, 13, and 14, as well as in Table 2.

**[Table 2]**

| Condition | Trehalose(-) | Trehalose(+) |
|---|---|---|
| Region | 1 | 2 |
| Raw Wells | 231,128 | **364,898** |
| Keypass Wells | 211,300 | **341,546** |
| Passed Filter Wells | 136,195 | **207,398** |

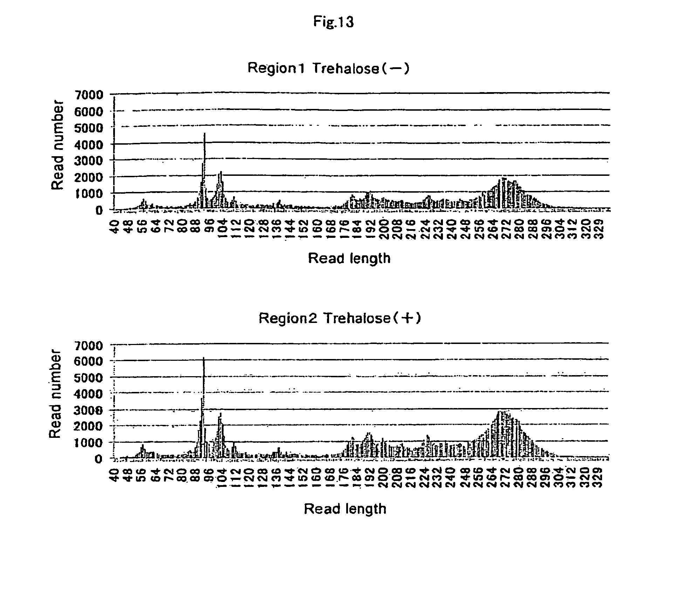

In emulsion PCR, trehalose (+) had the effect of increasing the DNA amplification wells compared to trehalose (-) (Fig. 12). Further, in the sequence analysis results, trehalose (+) had the effect of increasing the number of sequences and the size of the regions that were decoded relative to trehalose (-) (Fig. 13).

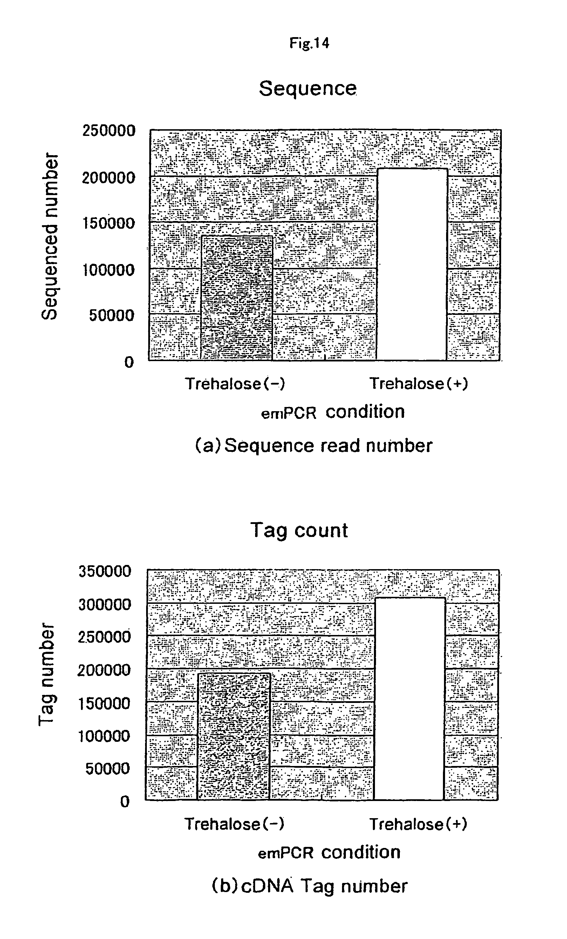

The number of sequences read in sample DNA when emulsion PCR was conducted with the addition of trehalose increased by about 1.6-fold relative to when no trehalose was added (Fig. 14(a)). Good results were also exhibited for the precision of the DNA fragments (tags) that were analyzed; more target regions were contained (Fig. 14(b)). Based on these results, trehalose affected the efficiency of enzymatic reactions (the DNA extension reaction) on reaction surfaces such as beads, and exhibited an effective property in promoting enzymatic activity. Such properties of trehalose can be expected to further enhance performance when applied to large-scale sequencing systems in which enzymatic reactions are conducted on supports.

### 12. Evaluation of the effect of trehalose on uniform liquid phase systems

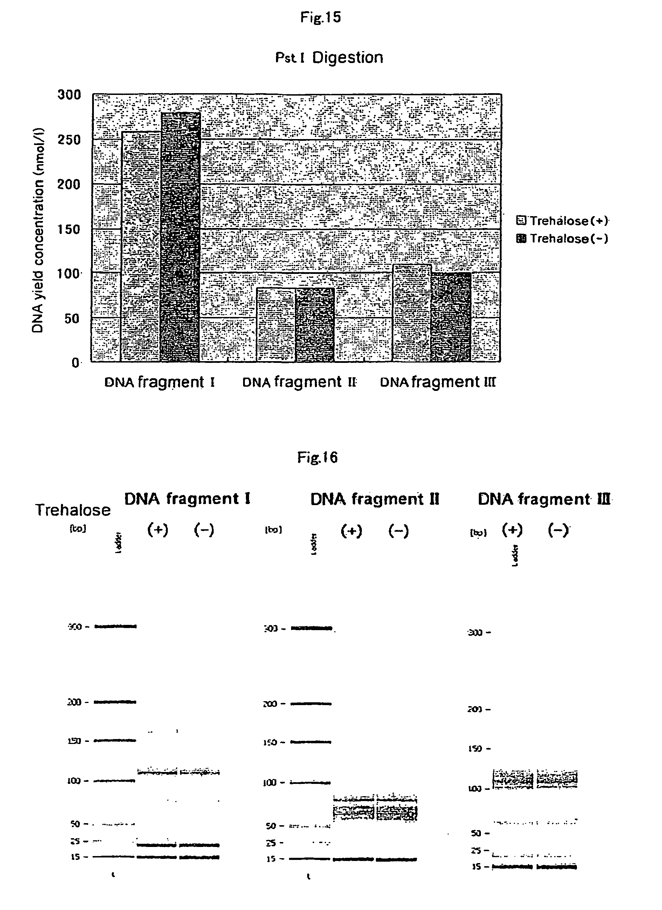

In the same manner as in 7 above, the three biotin-labeled DNA fractions I, II, and III (Seq. ID Nos. 4 to 6) were each cleaved at the restriction enzyme PstI site and the DNA fragments of various sizes that were released were detected. Two types of reaction solution (1x Pstl buffer, 1x BSA, restriction enzyme 40 units) were prepared: a trehalose (+) system with a final D(+)-trehalose concentration of 0.6 M, and a trehalose (-) system to which no trehalose was added. Each of the reaction solutions was admixed and reacted for three hours at 37°C. Cleavage was conducted with restriction enzyme. The DNA fragments of various sizes that were released were reacted with proteinase K, treated with phenol chloroform, and precipitated from ethanol. The precipitate of DNA fragments obtained was dissolved in 1x TE (pH 7.5) and analyzed with Bioanalyzer DNA 1000 assay (Agilent Technologies) (Figs. 15 and 16).

As a result, there was no change in the quantity of the released DNA fragments that were recovered based on whether or not trehalose was present (Figs. 15 and 16). In combination with the results of 7 above, these results clearly indicated that the addition of trehalose either increased the reactivity of the enzyme on the target DNA immobilized on the support, or reduced or suppressed inhibition by the support of the reactivity of the enzyme to the target substance.

### 13. Evaluation of the effect of added reagents on DNA fragments in the presence of saccharides, amino acids, polyhydric alcohols, and combinations thereof DNA fragment HindIII cleavage reaction on a spherical bead surface under conditions where additional substances such as D(+)-trehalose were present

As in 8 above, biotin-labeled DNA fragment I (Seq. ID No. 4, Fig. 30) was subjected to a HindIII cleavage reaction on the surface of beads with the addition of trehalose, sorbitol, glucose, betaine, ethylene glycol, or glycine. The 104 bp DNA fragments that were released were detected. Paramagnetic streptavidin beads were washed with accessory 1x binding buffer, recovered with a magnetic stand to remove the supernatant, and suspended in the same buffer. An equal volume of 2x binding buffer was admixed to DNA fragment I (1 microgram), the bead solution was added, and the mixture was gently stirred and adsorbed with top to bottom inversion and rotation for 20 minutes at room temperature. The sample-adsorbed beads were recovered with the magnetic stand, washed three times with 1x binding buffer, and washed once more with 1x HindIII buffer. A total of 23 reaction solutions (1x HindIII buffer, restriction enzyme 40 units, and various added reagents in the various concentrations indicated below) were prepared. The various reaction solutions were respectively added to washed beads and reacted for three hours at 37°C. The beads were recovered with the magnetic stand and cleaved with restriction enzyme. The supernatant containing the DNA of various sizes that was released was precipitated from ethanol. The precipitate of DNA fragments that was obtained was dissolved in 1x TE (pH 7.5) and analyzed with Bioanalyzer DNA 1000 assay (Agilent Technologies).

The various final concentrations of the added reagents were adjusted to: 0.1, 0.3, 0.5, and 0.6 M trehalose; 0.6 M sorbitol; 0.1, 0.3, 0.6, and 0.8 M glucose; 0.5 M betaine; 0.3 M ethylene glycol; 0.5 M glycine + 0.5 M betaine; 0.6 M trehalose + 0.6 M sorbitol; 0.3 M trehalose + 0.3 M sorbitol; 0.6 M trehalose + 0.6 M glucose; 0.3 M trehalose + 0.3 M glucose; 0.5 M trehalose + 0.5 M betaine; 0.3 M trehalose + 0.3 M betaine; 0.5 M trehalose + 0.5 M glycine; 0.3 M trehalose + 0.3 M glycine; 0.5 M trehalose + 0.5 M glycine + 0.5 M betaine; and 0.3 M trehalose + 0.3 M glycine + 0.3 M betaine.

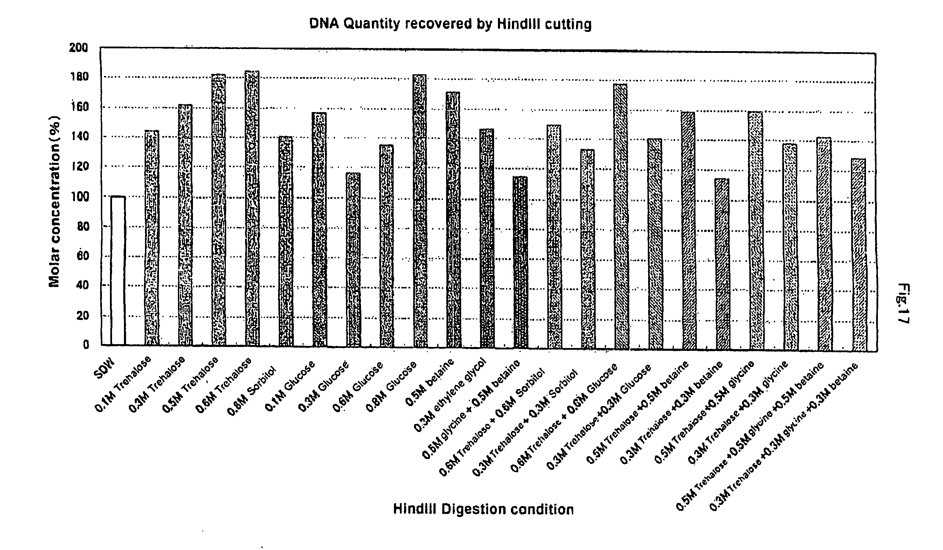

Fig. 17 shows the results of the above analysis. The quantity of DNA fragments recovered when the various concentrations of added reagents and their combinations were employed was in all cases greater than when they were not added.

### 14. Evaluation of the effect of trehalose in a DNA sequencing system accompanied by emulsion PCR (2)

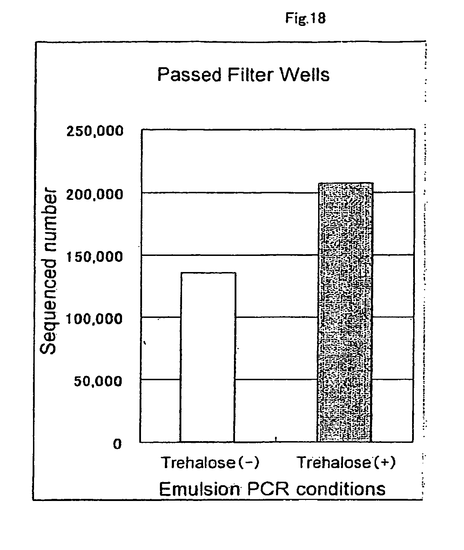

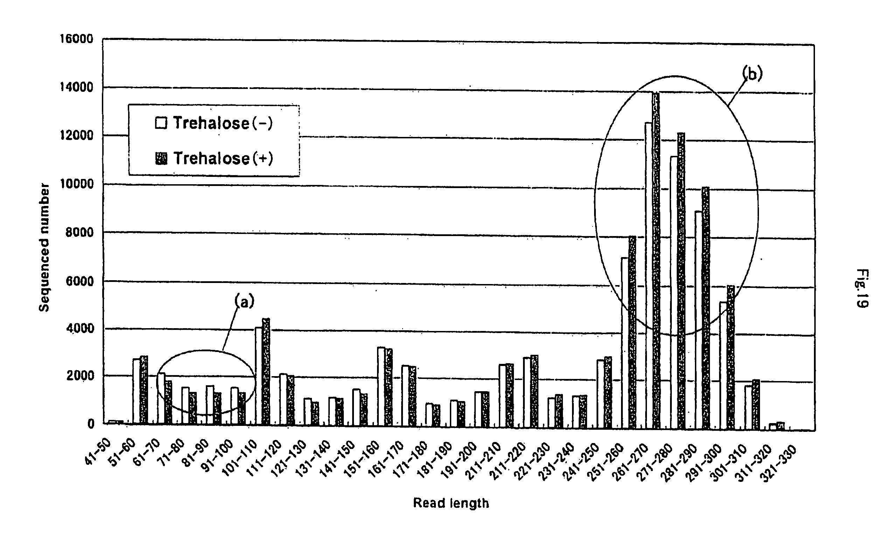

In the 454 sequencing system described in 11 above, trehalose was added to a final concentration of 0.3 M to the reaction solution, an amplification reaction was conducted, and the effect on the level of sequencing was examined. The results are given in Figs. 18 and 19.

As shown in Fig. 18, the fact that the addition of trehalose increased the number of sequences indicated that the addition of trehalose further increased the number of DNA fragments amplified by emulsion PCR, that is, trehalose increased the enzymatic activity in emulsion PCR.

In emulsion PCR, there is a problem in that short fragments end up increasing preferentially over long fragments. However, a look at the effect of the addition of trehalose on sequence length (see Fig. 19) reveals that the addition of trehalose resulted in a reduction in short fragments (a) and an increase in long fragments (b). That is, the addition of trehalose was found to afford improvement with regard to the above problem in emulsion PCR.

### 15. Evaluation of the effect of trehalose on a DNA sequencing system on a DNA chip (2)

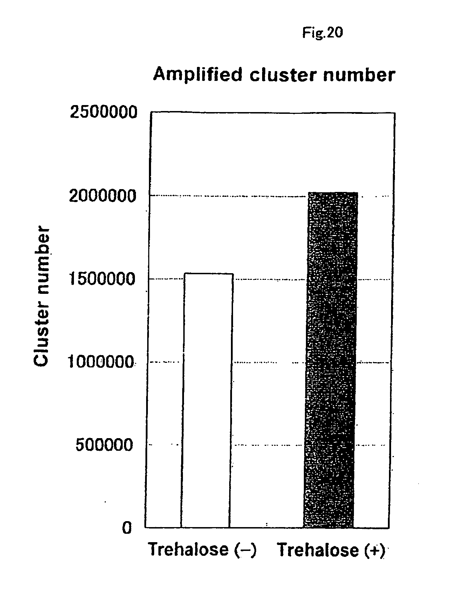

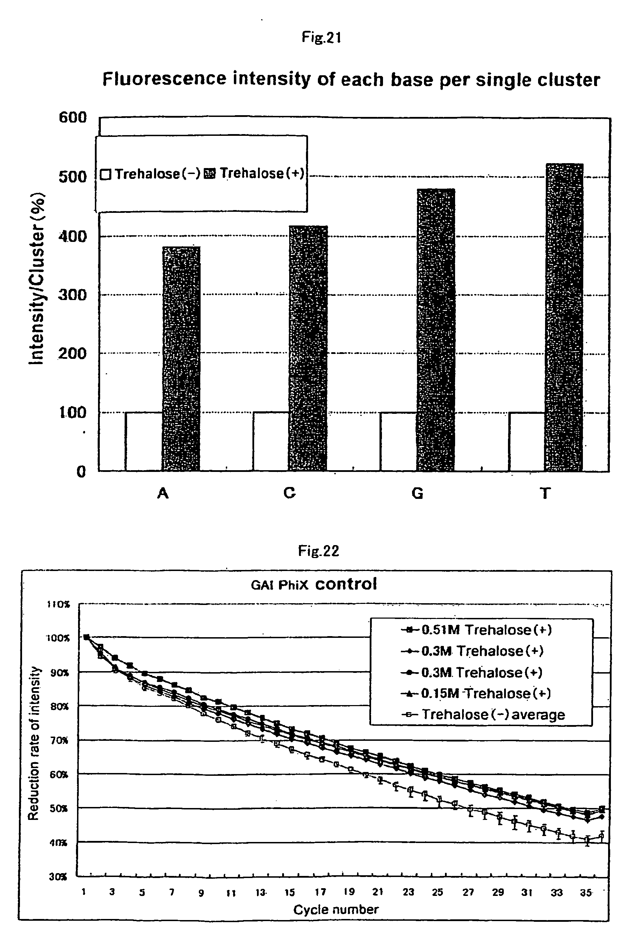

In the same manner as in 10 above, trehalose was added to a final concentration of 0.3 M to a reaction solution and amplification was conducted in a Solexa sequencing system. The results were compared to those obtained when no trehalose was added. The samples employed, prepared by the CAGE method (obtained by cleaving just 27 bp off the 5' end of the cDNA), were different 27 bp DNA fragments than those in 10 above. The results obtained by this sequencing system are given in Figs. 20 and 21.

The sequence level increased in the cluster amplification reaction conducted with the addition of trehalose (see Fig. 20). In the sequencing reaction following the cluster amplification reaction, each base in the CAGE samples obtained under conditions where trehalose was added exhibited a higher level of fluorescence intensity (see Fig. 21). These results indicated that the addition of trehalose increased the enzymatic activity (here, DNA extension activity) on a horizontal surface.

### 16. Evaluation of the effect of trehalose on the sequencing reaction in a Solexa sequencing system

### (1) Method

D(+)-trehalose was added to the enzyme reaction solution in a Genome Analyzer system (GAI) and the results were measured. The experiment was conducted with the three D(+)-trehalose final concentrations of 0.15 M, 0.3 M, and 0.51 M. The ratio of the reduction in the average value obtained by dividing the fluorescence intensity calculated for each cycle by the number of clusters was employed for comparison.

### (2) Materials

The D(+)-trehalose employed was D(+)-trehalose (90208 Biochemika, ≥ 99.5% (HPLC) (Fluka). A 36-cycle Illumina Sequencing Kit (FC-204-2036) was employed for sequencing with a Genome Analyzer (Illumina). The sample employed was PhiX control (CT-901-1001).

### (3) D(+)-trehalose addition conditions

The IMX36 (42 mL) in box 2 of the SBS Kit contained in the 36-Cycle SBS Reagent Kit v2 (Illumina catalog #FC-204-2036) was melted, after which D(+)-trehalose was added in the quantities indicated in Table 3.

**[Table 3]**

| Final concentration | Added quantity |
|---|---|
| 0.51 M | 9.06g |
| 0.3 M | 5.11g |
| 0.15 M | 2.40g |

Following the addition, the mixture was shielded from light and gently mixed with top to bottom inversion for 10 minutes at room temperature. Once the dissolution had been visually confirmed, the mixture was passed through a filter (with a filter pore system of 0.22 micrometers). Precisely the required 42 mL was collected from the mixed solution following filtration. Subsequently, the operations specified in the manufacturer's protocol were conducted.

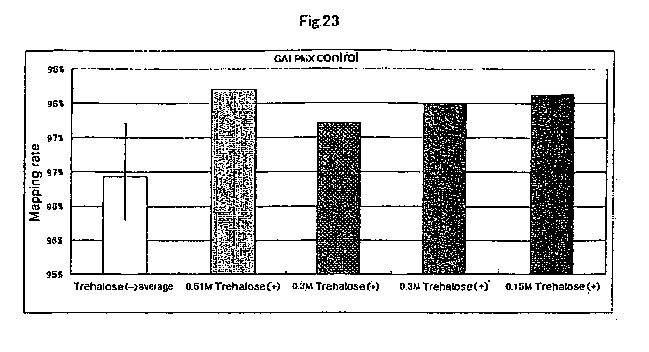

### (4) Results (Figs. 22 and 23)

Fig. 22 shows the average value calculated for the PhiX control of each run. As indicated in Fig. 22, a significant overall difference was exhibited under a trehalose (+) condition in the rate of reduction in the fluorescence intensity of the trehalose (-) and trehalose (+) sequences. The difference was about 20% or more compared to the 35th cycle, where the fluorescence intensity was at its weakest. Max and Min values are given for the trehalose (-) error bar to indicate the degree of fluctuation. Fig. 23 shows the PhiX control mapping rate. Here, as well, an overall significant difference was exhibited relative to trehalose (-).

Based on these results, enzymatic activity increased in the presence of trehalose due to the lower reduction rate in fluorescence intensity, and more molecular extension reactions were thought to occur than for trehalose (-). As a result, the mapping rate improved and the enzymes were activated.

### 17. Evaluation of the effect of trehalose on the sequencing reaction in a SOLiD sequencing system

### (1) Method

D(+)-trehalose was added to the ligation enzymatic reaction solution of a SOLiD system 2.0 and the effects were measured. The experiment was conducted at a final D(+)-trehalose concentration of 0.3 M. The noise to signal ratio (N/S ratio) of each cycle was employed for comparison. The N/S ratio was obtained by dividing the second brightest fluorescence intensity by the brightest fluorescence intensity.

### (2) Materials

The D(+)-trehalose employed was D(+)-trehalose dihydrate (201-02253 reagent grade) (Wako). A SOLiD^{(™)} fragment library sequencing kit v2 (4400466) was employed for sequencing with the SOLiD system (Applied Biosystems). The sample employed as a SOLiD^{(™)} DH10B fragment library control kit (4391889).

### (3) D(+)-trehalose addition conditions

Probe Mixes A and B and bridge probe, prepared with the reagents contained in a SOLiD^{(™)} fragment library sequence kit v2 (4400466) and adjusted to a D(+)-trehalose (90208 BioChemika, ≥ 99.5% (HPLC)) (Fluka) concentration of 1.7 M, were added to each sequence primer in the quantities indicated below.

**[Table 4]**

| Reagent | Added quantity |
|---|---|
| Probe Mix A | 67.5ul |
| Probe Mix B | 67.5ul |
| Bridge Probe | 13.5ul |

The operation was conducted according to the manufacturer's protocol following addition.

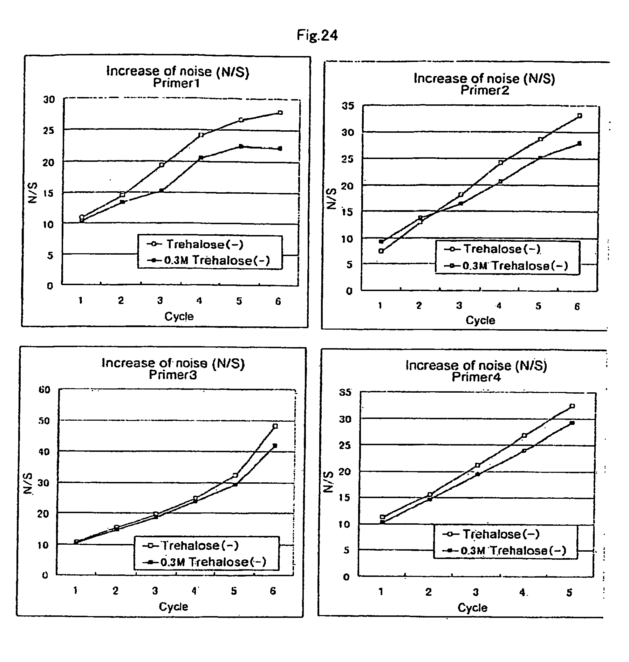

In the SOLiD sequencing kit, there was a problem in the form of increased noise with each cycle. However, as shown in Fig. 24, the addition of trehalose reduced the rise in noise, affording improvement with respect to this problem.

### 18. Evaluation of the effect of trehalose in the sequencing reaction of the 454 sequencing system

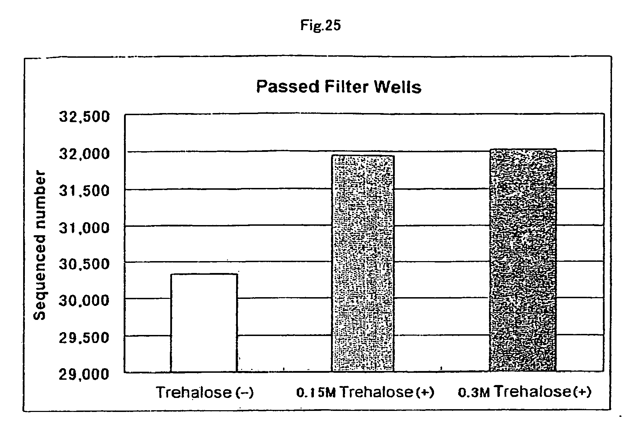

In the 454 sequencing system described in 11 above, trehalose was added to the reaction solution to a final concentration of 0.3 M, the sequencing reaction was conducted, and the effect on the sequencing level was observed. The results are given in Fig. 25.

### (1) Method

D(+)-trehalose was added to the sequencing enzyme reaction solution of a Genome Sequencer FLX system and the effects were measured. Two experiments were conducted with final D(+)-trehalose concentrations of 0.15 M and 0.3 M. The ratio of the read length to the decodable sample was employed for comparison.

### (2) Materials

The D(+)-trehalose employed was D(+)-trehalose dihydrate (201-02253 reagent grade) (Wako). A GS LR 25 Sequencing Kit (502906) was employed for sequencing with the Genome Sequencer FLX system. The samples employed here were a PolyA minus full length cDNA library prepared in-house and human genome prepared according to the manufacturers protocol.

### (3) D(+)-trehalose addition conditions

The D(+)-trehalose dihydrate (201-02253 reagent grade) (Wako) was added in the following proportion to the Buffer CB among the reagents contained in the GS LR 25 Sequencing Kit (502906).

**[Table 5]**

| Final concentration | Added quantity |
|---|---|
| 0.15 M | 121.71g |
| 0.3 M | 262.04g |

The mixture was stirred with a magnetic stirrer. Once dissolution had been visually confirmed, the mixed solution was passed through a filter (filter pore system 0.22 micrometer). Following filtration, precisely the required 2x 1000 mL quantities were collected from the mixed solution. Subsequent operations were carried out according to the manufacturers protocol.

As shown in Fig. 25, the addition of trehalose increased the number of sequences. This indicated that the addition of trehalose increased enzymatic activity in the sequence extension reaction, making it possible to decode more sequences.

## Claims

1. A method for reducing or suppressing the inhibitory effect of a support, on which a target substance is immobilized, on the reactivity of an enzyme to the target substance by placing the enzyme in the presence of one or more substances selected from the group consisting of saccharides, amino acids, polyhydric alcohols.

2. The method according to claim 1, wherein the sugar is selected from the group consisting of trehalose, maltose, glucose, sucrose, lactose, xylobiose, agarobiose, cellobiose, levanbiose, quitobiose, 2-β-glucuronosylglucuronic acid, allose, altrose, galactose, gulose, idose, mannose, talose, sorbitol, levulose, xylitol and arabitol.

3. The method according to claim 1 or 2, wherein the amino acid is selected from the group consisting of N^{a}-acetyl-β-lysine, alanine, *gamma*-aminobutyric acid, betaine, glycine betaine, N^{a}-carbamoyl-L-glutamine-1-amide, choline, dimethylthetine, ecotine, glutamate, β-glutamine, glycine, octopine, proline, sarcosine, taurine, and trimethylamine N-oxide.

4. The method according to any one of claims 1 to 3, wherein the target substance is a nucleic acid.

5. The method according to claim 4, wherein the nucleic acid is a single-stranded or double-stranded DNA or RNA.

6. The method according to claim 4, wherein the nucleic acid is comprised of hybridized DNA and RNA.

7. The method of any one of claims 1 to 6, wherein the enzyme is one or more enzymes selected from the group consisting of transferase, hydrolase, and synthase.

8. The method according to any one of claims 1 to 6, wherein the enzyme is DNA polymerase, RNase, DNA ligase and reverse transcriptase.

9. The method according to any one of claims 1 to 6, wherein the enzyme is a restriction enzyme.

10. The method according to any one of claims 1 to 9, wherein the support is a bead-like support or a plate-like support.

11. The method according to claim 10, wherein the bead-like support is streptavidin beads.

12. The method according to claim 10, wherein the plate-like support is streptavidin plates or DNA microarray plates.

13. The method according to any one of claims 1 to 12, wherein the method is employed in cDNA library preparation, a sequencing reaction, a DNA synthesis reaction, or GSC.

14. The method according to claim 13, wherein the DNA synthesis reaction is emulsion PCR or bridge PCR.

## Patentansprüche

1. Ein Verfahren zur Verringerung oder Unterdrückung der hemmenden Wirkung eines Trägers, auf dem eine Zielsubstanz immobilisiert ist, auf die Reaktivität eines Enzyms gegenüber der Zielsubstanz, indem das Enzym der Gegenwart von einer Substanz oder von mehreren Substanzen ausgesetzt wird ausgewählt aus der Gruppe bestehend aus Sacchariden, Aminosäuren, mehrwertigen Alkoholen.

2. Das Verfahren gemäß Anspruch 1, wobei der Zucker ausgewählt ist aus der Gruppe bestehend aus Trehalose, Maltose, Glucose, Saccharose, Lactose, Xylobiose, Agarobiose, Cellobiose, Lävanbiose, Chitobiose, 2-β-Glucuronosylglucuronsäure, Allose, Altrose, Galactose, Gulose, Idose, Mannose, Talose, Sorbit, Lävulose (Fructose), Xylit und Arabit.

3. Das Verfahren gemäß Anspruch 1 oder 2, wobei die Aminosäure ausgewählt ist aus der Gruppe bestehend aus N^{e}-Acetyl-β-Lysin, Alanin, *gamma-*Aminobuttersäure, Betain, Glycinbetain, N^{a}-Carbamoyl-L-Glutamin-1-Amid, Cholin, 2-Butin (Dimethylthäthin), Ecotin, Gutamat, β-Glutamin, Glycin, Octopin, Prolin, Sarkosin, Taurin und Trimethylamin-N-oxid.

4. Das Verfahren gemäß irgend einem der Ansprüche 1 bis 3, wobei die Zielsubstanz eine Nukleinsäure ist.

5. Das Verfahren gemäß Anspruch 4, wobei die Nukleinsäure eine einzelsträngige oder doppelsträngige DNA oder RNA ist.

6. Das Verfahren gemäß Anspruch 4, wobei die Nukleinsäure hybridisierte DNA und RNA enthält.

7. Das Verfahren gemäß irgend einem der Ansprüche 1 bis 6, wobei das Enzym ein Enzym oder mehrere Enzyme darstellt ausgewählt aus der Gruppe bestehend aus Transferase, Hydrolase und Synthase.

8. Das Verfahren gemäß irgend einem der Ansprüche 1 bis 6, wobei das Enzym DNA-Polymerase, RNase, DNA-Ligase und Reverse Transkriptase ist.

9. Das Verfahren gemäß irgend einem der Ansprüche 1 bis 6, wobei das Enzym ein Restriktionsenzym ist.

10. Das Verfahren gemäß irgend einem der Ansprüche 1 bis 9, wobei der Träger ein perlenartiger Träger oder ein plattenartiger Träger ist.

11. Das Verfahren gemäß Anspruch 10, wobei der perlenartige Träger Streptavidin-gekoppelte Perlen darstellt.

12. Das Verfahren gemäß Anspruch 10, wobei der plattenartige Träger Streptavidin-gekoppelte Platten oder DNA-Microarray-Platten darstellt.

13. Das Verfahren gemäß irgend einem der Ansprüche 1 bis 12, wobei das Verfahren angewandt wird bei der Herstellung von cDNA-Bibliotheken, bei einer Sequenzierungsreaktion, bei einer DNA-Synthesereaktion oder einer Klonierung mittels Gensignatur (GSC).

14. Das Verfahren gemäß Anspruch 13, wobei die DNA-Synthesereaktion eine Emulsions-PCR oder eine Brücken-PCR ist.

## Revendications

1. Procédé de réduction ou de suppression de l'effet inhibiteur d'un support, sur lequel une substance cible est immobilisée, sur la réactivité d'une enzyme à la substance cible en plaçant l'enzyme en présence d'une ou plusieurs substances choisies dans le groupe consistant en les saccharides, les acides aminés et les alcools polyhydriques.

2. Procédé selon la revendication 1, dans lequel le sucre est choisi dans le groupe consistant en le trehalose, le maltose, le glucose, le saccharose, le lactose, le xylobiose, l'agarobiose, le cellobiose, le lévanbiose, le quitobiose, l'acide 2-β-glucuronosylglucuronique, l'allose, l'atrose, le galactose, le gulose, l'idose, le mannose, le talose, le sorbitol, le lévulose, le xylitol et l'arabitol.

3. Procédé selon la revendication 1 ou 2, dans lequel l'acide aminé est choisi dans le groupe consistant en la N'-acétyl-β-lysine, l'alanine, l'acide *gamma*-aminobutyrique, la bétaïne, la glycine bétaïne, le N^{a}-carbamoyl-L-glutamine-1-amide, la choline, la diméthylthétine, l'écotine, le glutamate, la β-glutamine, la glycine, l'octopine, la proline, la sarcosine, la taurine et la triméthylamine N-oxyde.

4. Procédé selon l'une quelconque des revendications 1 à 3, dans lequel la substance cible est un acide nucléique.

5. Procédé selon la revendication 4, dans lequel l'acide nucléique est un ADN ou ARN simple brin ou double brin.

6. Procédé selon la revendication 4, dans lequel l'acide nucléique est composé d'ADN et d'ARN hybridés.

7. Procédé selon l'une quelconque des revendications 1 à 6, dans lequel l'enzyme est une ou plusieurs enzymes choisies dans le groupe consistant en une transférase, une hydrolase et une synthase.

8. Procédé selon l'une quelconque des revendications 1 à 6, dans lequel l'enzyme est l'ADN polymérase, la RNase, l'ADN ligase et la transcriptase inverse.

9. Procédé selon l'une quelconque des revendications 1 à 6, dans lequel l'enzyme est une enzyme de restriction.

10. Procédé selon l'une quelconque des revendications 1 à 9, dans lequel le support est un support semblable à une bille ou un support semblable à une plaque.

11. Procédé selon la revendication 10, dans lequel le support semblable à une bille est constitué de billes de streptavidine.

12. Procédé selon la revendication 10, dans lequel le support semblable à une plaque est constitué de plaques de streptavidine ou de plaques de microréseau à ADN.

13. Procédé selon l'une quelconque des revendications 1 à 12, dans lequel le procédé est employé dans une préparation de banque d'ADNc, une réaction de séquençage, une réaction de synthèse d'ADN ou un clonage par signature génétique (GSC).

14. Procédé selon la revendication 13, dans lequel la réaction de synthèse d'ADN est une amplification en chaîne par polymérase (PCR) en émulsion ou une PCR pont.


### SEQUENCE LISTING

<110> RIKEN
<120> Method for enhancement of enzyme reactions
<130> A95039H
<160> 6
<170> PatentIn version 3.4
<210> 1
   <211> 24
   <212> DNA
   <213> primer01
<400> 1
   ctcactaaag ggaacaaaga tgtg 24
<210> 2
   <211> 24
   <212> DNA
   <213> primer02
<400> 2
   cattaatgca gctggcacga cagg 24
<210> 3
   <211> 51
   <212> DNA
   <213> Artificial sequence
<220>
   <223> primer
<400> 3
   aaagaattcg cggccgcaag cttctgcagc tcgagttttt tttttttttt t 51
<210> 4
   <211> 135
   <212> DNA
   <213> DNA fragmentI
<400> 4
   ttttgaggtg cggttgctgg cgcctctgca ggggctcgcc acttcgggct cggatccgcc 60
   ggcatcaccg gcgccacctc gagctcatca ttggaaaacg ttcgaattca agctttgagg 120
   cacctatctc agcga 135
<210> 5
   <211> 135
   <212> DNA
   <213> DNA fragment II
<400> 5
   ttttgatgag cgcttgtttc ggcgtctcga gtcatggcga ccacacccgt cggatccaaa 60
   ctctcaagga tcttaccctg cagcagcatc ttttactttc accgaattca agctttgagg 120
   cacctatctc agcga 135
<210> 6
   <211> 135
   <212> DNA
   <213> DNA fragment III
<400> 6
   ttttgatgag cgcttgtttc ggcgtctcga gtcatggcga ccacacccgt cggatccagc      60
   gtttctgggt gagcaaaaag cttaacagga aggcaaaatg ccggaattcc tgcagaataa     120
   gggcgacacg gaaat                                                       135